# EUROPEAN PATENT SPECIFICATION

(11) **EP 4 622 599 B1**
(45) Date of publication and mention of the grant of the patent: **18.11.2026**
(21) Application number: 23829219.7
(22) Date of filing: 22.11.2023
(51) Int. Cl.: A61F 2/24, A61F 2/95

(54) **CATHETER SYSTEM FOR SEQUENTIAL DEPLOYMENT OF AN EXPANDABLE IMPLANT**
KATHETERSYSTEM ZUM SEQUENTIELLEN EINSATZ EINES EXPANDIERBAREN IMPLANTATS
SYSTÈME DE CATHÉTER POUR LE DÉPLOIEMENT SÉQUENTIEL D'UN IMPLANT EXPANSIBLE

(30) Priority: 23.11.2022 US 202263384843 P
(43) Date of publication of application: 01.10.2025
(73) Proprietor: JenaValve Technology, Inc., Irvine, CA 92618 (US)
(72) Inventor: LEE, Rolando, Irvine, California 92618 (US); CHU, Kevin, Tustin, California 92782 (US); CORRADO, Michael, Laguna Hills, California 92653 (US); MIRIGIAN, Greg, Tustin, California 92783 (US); BUJURU, Karthik, Aliso Viejo, California 92656 (US)
(74) Representative: Meissner Bolte Partnerschaft mbB
(86) International application number: PCT/US2023/080979
(87) International publication number: WO 2024/112929

(56) References cited:
- WO-A1-2021/242607
- US-A1- 2017 325 954
- US-A1- 2022 061 987

## Description

### CROSS-REFERENCE TO RELATED APPLICATIONS

This application claims the benefit of U.S. Provisional Patent Application Serial No. 63/384,843, filed November 23, 2022.

### TECHNICAL FIELD

The present invention relates, in general, to catheter systems for deploying a cardiac implant. For example, systems and methods are provided herein including a catheter system for precise, sequential placement of self-expanding prosthetic heart valves.

### BACKGROUND

The document US 2022/0061987 A1 relates to a delivery system for prosthetic medical devices. The delivery system is configured to cause relative motion between a delivery capsule and a prosthetic device within the delivery capsule. The delivery system includes a first fluid pathway and a second fluid pathway and may be configured to switch between the first fluid pathway and the second fluid pathway as the delivery system transitions toward a deployment configuration. The document US 2017/0325954 A1 relates to a delivery device for a collapsible prosthetic heart valve that includes a catheter assembly and an operating handle. The document WO 2021/242607 A1 relates to a delivery device for use in delivering a prosthesis via percutaneous transcatheter techniques.

In medical technology, there has been an endeavor to treat or fix a heart valve defect, such as an aortic valve insufficiency or an aortic valve stenosis, non-surgically using transarterial interventional access via a catheter, thus without an invasive surgical operation. Transcatheter aortic valve replacement (TAVR) and transcatheter aortic valve intervention (TAVI) procedures are becoming more commonplace. Various insertion systems and stent systems have been proposed, with different advantages and disadvantages, which in part can be introduced into the body of a patient transarterially by means of a catheter system.

In the medical devices previously known, however, it has become apparent that the implantation procedure of a stent system to which the heart valve prosthesis is attached is relatively complicated, difficult, and expensive. Apart from the complicated implantation of the heart valve prosthesis as a replacement for an insufficient or defective native heart valve, there is the fundamental risk of incorrect positioning of the stent or heart valve prosthesis with the medical devices used up to the present, which cannot be corrected without more extensive and invasive surgical intervention.

It is also regarded as problematic that, when using systems already known from the state-of-the-art, incorrect positioning of the heart valve prosthesis or the associated heart valve stent can often only be avoided when the heart surgeon or interventional cardiologist is especially experienced.

Improved delivery catheter systems are described in, for example, U.S. Patent Application No. 18/504,932, U.S. Patent No. 11,065,138 to Schreck, U.S. Patent No. 11,147,669 to Straubinger, and U.S. Patent No. 8,679,174 to Ottma.

What is needed are further improved systems and devices for introducing a sequentially expandable heart valve stent into the body of a patient, for positioning the stent at a desired implantation site, and for reducing the risk to the patient on implantation.

### SUMMARY

The present invention relates to a catheter system according to independent claim 1, wherein further developments of the inventive catheter system are provided in the sub-claims, respectively.

Provided herein are catheter systems and methods for implanting a prosthetic heart valve in a sequential manner. The catheter system may be used to implant a self-expandable prosthetic heart valve having arms that allow the prosthetic heart valve to clip onto the native valve. This method of attachment allows the valve to be placed into the heart without having to sew the prosthetic valve into the heart. The delivery catheter described herein may be used to sequentially deploy the prosthetic valve. The delivery catheter may be capable of positioning the prosthetic valve over or near the native valve and releasing the portions of the prosthetic valve sequentially from the delivery catheter. The delivery catheter allows for precise positioning of the prosthetic valve at a desired implantation site. The prosthetic valve can then be sequentially deployed in a controlled manner for improved precision and safety.

A catheter system for implanting a prosthetic heart valve may, in one example, include an elongated shaft having a proximal region and a distal region, a deployment assembly at the distal region of the elongated shaft, the deployment assembly sized and shaped to be advanced to an implantation site at a native heart valve site with the prosthetic heart valve in a collapsed state, the deployment assembly including a sleeve designed to maintain at least a portion of the prosthetic heart valve in the collapsed state within the sleeve during delivery, a handle positioned at a proximal region of the elongated shaft, the handle having a handle body, a first actuator designed to be rotated relative to the handle body, and a second actuator designed to transition between a first position and a second position. When the second actuator is in the first position, rotation of the first actuator may be translated to the deployment assembly to cause the deployment assembly to rotate and, when the second actuator is in the second position, rotation of the first actuator may cause the sleeve to move longitudinally relative to the handle for expanding and implanting the prosthetic heart valve.

The handle may have a third actuator that, when in a first position, prevents movement of the first actuator when the second actuator is in the first position and, when in a second position, permits movement of the first actuator when the second actuator is in the first position. The handle may include a fourth actuator designed to rotate relative to the handle body and independent of the first actuator, the fourth actuator may be connected to a distal portion of the elongated shaft and designed to cause the elongated shaft to deflect. When the second actuator is in the second position, rotation of the first actuator may not cause the deployment assembly to rotate and, when the second actuator is in the first position, rotation of the first actuator may not cause the sleeve to move longitudinally relative to the handle.

The deployment assembly may include a second sleeve proximal to the sleeve and coupled to the elongated shaft and an anchor support positioned within the second sleeve and in mechanical communication with the sleeve. The anchor support may be designed to receive a proximal portion of the prosthetic valve and the anchor support and the second sleeve may be designed to retain the proximal portion of prosthetic heart valve in a compressed state. Longitudinal movement of the sleeve may cause longitudinal movement of the anchor support. When the second actuator is in the first position, the first actuator may be rigidly connected to the deployment assembly. The second actuator may include a protrusion and when the second actuator is in the first position, the protrusion of the second actuator may be designed to engage a shaft positioned within the handle and rigidly connected to the deployment assembly.

The shaft may be threaded and a cross-section of the shaft may include at least one right angle. The handle may include a third actuator designed to interface with one or more threads of the shaft and the at least one right angle to selectively restrain the deployment assembly from axial and rotational movement. The third actuator may include a depressible body having a central channel and a ridged wheel positioned within the central channel and designed to receive and rotate with the shaft, and wherein the third actuator is designed to resist rotation of the ridged wheel in a locked position.

In accordance with another aspect, a method for implanting a prosthetic heart valve is provided. The method may include advancing a deployment assembly at a distal region of an elongated shaft to an implantation site at a native heart valve site with the prosthetic heart valve in a collapsed state, the deployment assembly comprising a sleeve configured to maintain at least a portion of the prosthetic heart valve in the collapsed state within the sleeve during delivery; rotating a first actuator of a handle coupled to the elongated shaft relative to a handle body of the handle while a second actuator of the handle is in a first position such that rotation of the first actuator is translated to the deployment assembly to cause the deployment assembly to rotate; transitioning the second actuator from the first position to a second position; and rotating the first actuator while the second actuator is in the second position to cause the sleeve to move longitudinally relative to the handle for expanding and implanting the prosthetic heart valve.

A catheter system for implanting a prosthetic heart valve may, in another example, include an elongated shaft having a proximal region and a distal region, a deployment assembly at the distal region of the elongated shaft, the deployment assembly sized and shaped to be advanced to an implantation site at a native heart valve site with the prosthetic heart valve in a collapsed state, the deployment assembly including a sleeve, an anchor support designed to be positioned within the sleeve during delivery to maintain at least a portion of the prosthetic heart valve in the collapsed state between the sleeve and the anchor support, and a lock designed to lock the sleeve to the anchor support during delivery, and a handle positioned at a proximal region of the elongated shaft, the handle designed to, when actuated, cause the lock of the deployment assembly to unlock such that the anchor support is longitudinally moveable relative to the sleeve for expanding and implanting the prosthetic heart valve.

The lock may include a protrusion designed to extend into a receptacle to lock the sleeve to the anchor support and may be designed to be released from the receptacle to unlock the sleeve from the anchor support. The anchor support may include the protrusion and the sleeve may include the receptacle. The lock may include a tube designed to, in a first position, cause the protrusion to extend into the receptacle, the tube designed to move relative to the sleeve to a second position to release the protrusion from the receptacle. The protrusion may include a ball bearing designed to be held in the receptacle when locked and designed to be released from the receptacle when unlocked. The protrusion may include a lever designed to be held in the receptacle in a snapping manner when locked and released from the receptacle when unlocked. The deployment assembly may include a second sleeve distal to the sleeve and the sleeve may be connected to at least a portion of the elongated shaft.

The second sleeve may be designed to receive a distal portion of the prosthetic valve and maintain at least a distal portion of the prosthetic heart valve in a collapsed state. The longitudinal movement of the second sleeve may cause longitudinal movement of the anchor support. The deployment assembly may further include a tube positioned within the anchor support and designed to move longitudinally within the anchor support between a first position and a second position distal to the first position. The tube may have a non-uniform outer diameter. The tube includes a protrusion extending from an outer surface of the tube designed to engage anchor support to cause anchor support to move with the tube when the protrusion engages the anchor support. The tube may be connected to the second sleeve via a cable designed to cause distal movement of the tube in response to distal movement of the second sleeve. The handle may include a handle body and an actuator designed to be rotated relative to the handle body, the actuator may be designed to cause the anchor support to move longitudinally relative to the sleeve.

In accordance with another aspect, a method for implanting a prosthetic heart valve using a catheter system is provided. The method may include guiding a deployment assembly loaded with the prosthetic heart valve in a collapsed state to an implantation site at a native heart valve site, the deployment assembly positioned at a distal region of an elongated shaft and comprising a sleeve, an anchor support configured to be disposed within the sleeve to maintain at least a portion of the prosthetic heart valve in the collapsed state between the sleeve and the anchor support, and a lock configured to lock the sleeve to the anchor support; and rotating an actuator of a handle positioned at a proximal region of the elongated shaft to cause a first shaft extending between the actuator and the deployment assembly to move distally to release the lock to permit the anchor support to move with respect to the sleeve for expanding the at least a portion of the prosthetic heart valve for implanting the prosthetic heart valve.

A catheter system for implanting a prosthetic heart valve may, in another example, include an elongated shaft including a proximal region and a distal region, the elongated shaft including a cut hypotube including a proximal portion, a transition portion cut to have greater flexibility than the proximal portion, and a distal portion cut to have greater flexibility than the transition portion, a deployment assembly at the distal region of the elongated shaft, the deployment assembly sized and shaped to be advanced to an implantation site at a native heart valve site with the prosthetic heart valve in a collapsed state, and a handle positioned at a proximal region of the elongated shaft, the handle designed to, when actuated, cause the deployment assembly to release the prosthetic heart valve for expanding and implanting the prosthetic heart valve.

The catheter system may further include a deflection cable. The elongated shaft may further include a deflection shaft coupled at a distal end to the deflection cable. The cut hypotube and the deflection cable may be positioned within the deflector shaft. The handle may further include a handle body and a deflection actuator in mechanical communication with the deflection cable and designed to cause deflection cable to retract proximally. The deflection actuator may be designed to cause the deflection shaft to deflect. The elongated shaft may include a torque shaft positioned within the deflection shaft and designed to translate axial and rotational movement from the handle to the deployment assembly. The torque shaft may include a second hypotube, a polymer layer positioned within the second hypotube, a braid layer positioned within the polymer layer, and a liner layer including a fluoropolymer positioned within the braid layer. The second hypotube may be cut to increase flexibility in the proximal to distal direction, the polymer layer may include a nylon polymer, the braid layer may include a metallic braid, and the liner layer may include polytetrafluoroethylene (PTFE).

The elongated shaft further may include a guidewire shaft designed to receive a guide wire and may be positioned within the torque shaft, the torque shaft and the guidewire shaft may be axially independent. The guidewire shaft may include the cut hypotube, a second polymer layer positioned within the hypotube, a second braid layer positioned within the second polymer layer, and a second liner layer including a fluoropolymer positioned within the second braid layer. The hypotube may be longer than and have a greater number of cuts than the second hypotube. One or more of the hypotube or second hypotube may be a laser cut hypotube or micro-machined.

In accordance with another aspect, a method for implanting a prosthetic heart valve is provided. The method may include advancing a deployment assembly at a distal region of an elongated shaft to an implantation site at a native heart valve site with the prosthetic heart valve in a collapsed state, the elongated shaft comprising a cut hypotube comprising a proximal portion, a transition portion cut to have greater flexibility than the proximal portion, and a distal portion cut to have greater flexibility than the transition portion; and actuating a handle disposed at a proximal region of the elongated shaft to cause the deployment assembly to release the prosthetic heart valve for expanding and implanting the prosthetic heart valve.

### BRIEF DESCRIPTION OF THE DRAWINGS

FIG. 1 illustrates an exemplary catheter system including a deployment assembly, an elongated shaft, and a handle, in accordance with some aspects of the present invention.
FIGS. 2A-2B illustrate perspective views of an exemplary handle of the delivery system.
FIGS. 3A-3F illustrate perspective and cross-sectional views of an exemplary deployment assembly and perspective views of an exemplary retraction taper assembly.
FIG. 4 illustrates a cross-sectional view of the layers of an exemplary elongate shaft.
FIG. 5 illustrates a cross-sectional view of the layers of an exemplary torque shaft.
FIG. 6 illustrates cross-sectional views of an exemplary torque shaft.
FIG. 7 illustrates a side view of an exemplary hypotube of the torque shaft.
FIG. 8 illustrates a cross-sectional view of the layers of an exemplary guidewire shaft.
FIG. 9 illustrates a cross-sectional view of the guidewire shaft.
FIG. 10 illustrates cross-sectional views of an exemplary hypotube of the guidewire shaft.
FIGS. 11A-11C illustrate side and cross-sectional views of an exemplary handle and positioning shaft.
FIGS. 12A-12C illustrate perspective views of an exemplary unlocking actuator assembly and positioning shaft.
FIGS. 13A-13B illustrate perspective views of an exemplary unlocking actuator assembly including a catch thread and an exemplary positioning shaft.
FIGS. 14A-14C illustrate a perspective views of an exemplary interior threaded ring and an exemplary support drum.
FIG. 15 illustrates a perspective exploded view of an exemplary positioning actuator.
FIGS. 16A-16C illustrate a cross-sectional views of the positioning actuator and an exemplary deployment base.
FIGS. 17A-17B illustrates perspective and cross-sectional views of the positioning actuator and the releasing actuator.
FIGS. 18A-18C illustrate perspective views of an exemplary deployment thread and deployment base.
FIG. 19 illustrates manipulation of the deflector actuator to cause the elongated shaft to deflect.
FIG. 20A illustrates manipulation of an exemplary positioning actuator to cause the deployment assembly to move rotationally and axially and FIG. 20B illustrates manipulation of an exemplary positioning actuator and releasing actuator to cause the distal sleeve and anchor support move axially.
FIGS. 21A-21B illustrate an exemplary introducer sheath and causing an exemplary deployment assembly to traverse the introducer sheath.
FIGS. 22A-22E illustrate manipulation of any exemplary deployment assembly to release an exemplary prosthetic heart valve.
FIGS. 23A-23B illustrate an exemplary deployment assembly with a proximal sleeve and anchor support.
FIGS. 24A-24E illustrates a perspective and cross-sectional views of an exemplary proximal sleeve and anchor support including an internal support and a lock.
FIGS. 25A-25D illustrates a perspective and cross-sectional views of an exemplary distal sleeve and anchor support including an internal support and a cantilevered lock.

### DETAILED DESCRIPTION

The present invention is directed to a catheter system for introducing a cardiac implant such as a prosthetic heart valve into the body of a patient. Specifically, the catheter system may include an elongated shaft having a distal end connected to a deployment assembly designed to secure the cardiac implant and a proximal end connected to a handle used for manipulating the deployment assembly to sequentially deploy the prosthetic heart valve. The elongated shaft may have one or more hyptotubes that may be laser cut, micro-machined, and/or cut using any other well-known technique. The prosthetic heart valve may be a self-expanding prosthetic heart valve. The handle may include a deflection actuator and a positioning actuator that may rotate independently. The deflection actuator may, when rotated, cause the elongated shaft to arch and/or bend. The positioning actuator may, when in a locked position, cause the deployment assembly to rotate and/or advance distally or withdraw proximally. In the unlocked position, the positioning actuator, when rotated, may cause the deployment assembly to sequentially release the prosthetic heart valve to permit the prosthetic heart valve to transition to an expanded state.

The delivery catheter described herein is particularly well-suited for sequential deployment of a self-expandable prosthetic heart valve with arms that may clip onto a native valve, such as those described in U.S. Patent No. 11,154,398 to Straubinger. delivery catheter may be capable of positioning the prosthetic heart valve over or near the native valve, releasing arms of the prosthetic heart valve thereby allowing for partial expansion of the prosthetic heart valve, aligning the arms with native cusps of the native valve, and permitting the prosthetic heart valve to fully expand by uncoupling the valve from the delivery catheter, thereby clipping the prosthetic heart valve onto the native valve leaflets. The prosthetic heart valve, once implanted, is designed to treat or fix a heart valve defect, such as aortic valve regurgitation, aortic valve insufficiency, and/or aortic valve stenosis. One or more components of the delivery catheter described herein may be made from plastic, metal, alloy, combined materials and/or any other well-known materials in in the field of transcatheter cardiovascular devices.

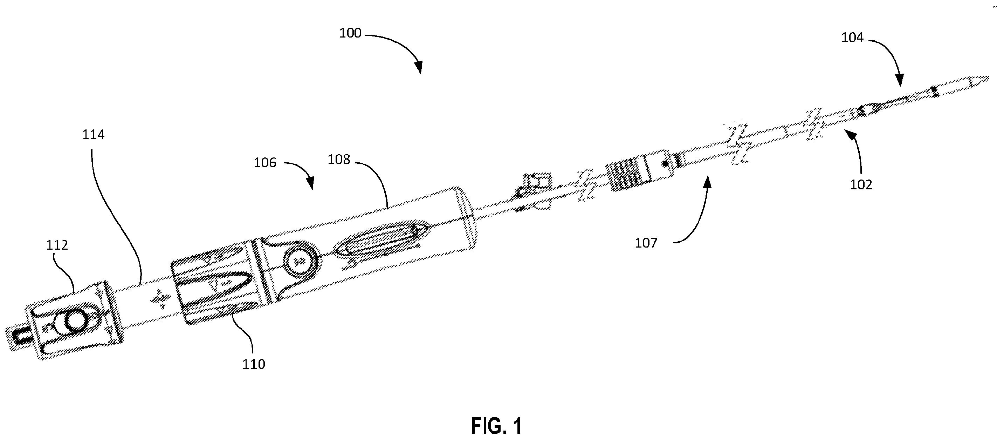

Referring now to FIG. 1, an exemplary catheter system for sequential deployment of a cardiac implant such as a prosthetic heart valve is illustrated. As shown in FIG. 1, catheter system 100 may include elongated shaft 102 with deployment assembly 104 at a distal end and a handle 106 at the proximal end. Elongated shaft 102 may house one or more tubes, cables and/or wires internally. For example, elongated shaft 102 may include concentric internal shafts that may move independently both rotationally and axially. In one example, an innermost internal shaft may be a guidewire shaft designed to permit passage of a guidewire. The guidewire shaft maybe positioned within a larger torque shaft, which may translate torque across elongate shaft 102. It is understood that deployment assembly 104 and at least a portion of elongate shaft 102 may be positioned into an introducer shaft for delivery to an implantation site.

Deployment assembly 104 may include sleeves and supports designed to at least partially house the prosthetic heart valve, maintain the prosthetic heart valve in at least a partially compressed state, and to permit the prosthetic heart valve to sequentially expand. For example, deployment assembly 104 may include an anchor support sized to receive a proximal end of the prosthetic heart valve and a proximal sleeve positioned over the anchor support to retain the proximal end of the prosthetic heart valve in the anchor support. Deployment assembly 104 may further include a distal sleeve designed to retain a distal end of the prosthetic heart valve. The anchor support and the distal sleeve may be designed to move sequentially to sequentially expand the prosthetic heart valve. The distal sleeve may be connected to the guidewire shaft and the distal sleeve may interface with the anchor support such that movement of the guidewire shaft may be translated to the distal sleeve and/or the anchor support.

Elongated shaft 102 may be coupled to handle 106 and interior components of elongated shaft 102 may be mechanically connected to handle 106. Handle 106 includes one or more actuators that, when actuated, cause a predetermined movement at deployment assembly 104. The actuators are preferably designed to interwork with one another to cause various actions during the deployment sequence. For example, a first actuator may be designed to cause one movement at the deployment assembly when a second actuator is in a first position and to cause a different movement at the deployment assembly when the second actuator is in a second position. The actuators may be knobs, buttons, switches, or the like suitable for use in a catheter system. Handle 106 may include handle body 108, deflection actuator 110, positioning actuator 112, and positioning shaft 114. Deflection actuator 110 may be connected to handle body 108 via a threaded connection and may rotate about the longitudinal axis of handle body 108. Deflection actuator 110 may be connected to a cable extending within elongated shaft 102 and coupled to a distal region of elongated shaft 102. As deflector actuator 110 is rotated about handle body 108, the cable may be retracted and may cause elongated shaft 102 to arch or bend.

Positioning actuator 112 is engaged with handle body 108 via positioning shaft 114 which is free to move into and out of and rotate with respect to handle body 108. Positioning actuator 112 is also designed to rotate with respect to a deployment base housed within positioning actuator 112. In a locked position, positioning actuator 112 may engage positioning shaft 114 and rotation of positioning actuator 112 may cause deployment assembly 104 to rotate and axial movement of positioning actuator 112 may result in axial movement of deployment assembly 104. In an unlocked position, positioning actuator 112 may disengage positioning shaft 114 such that rotation of positioning actuator 112 may cause deployment base to advance, causing the distal sleeve and/or anchor support of deployment assembly 104 to move distally, thereby sequentially expanding and releasing the prosthetic heart valve. Catheter system 100 may be combined with introducer sheath 107. For example, introducer sheath may have a larger diameter than elongated shaft 102 and deployment assembly 104 and may include a handle at a proximal end. Introducer sheath 107 may also be combined with a dilator at a distal end of introducer sheath 107.

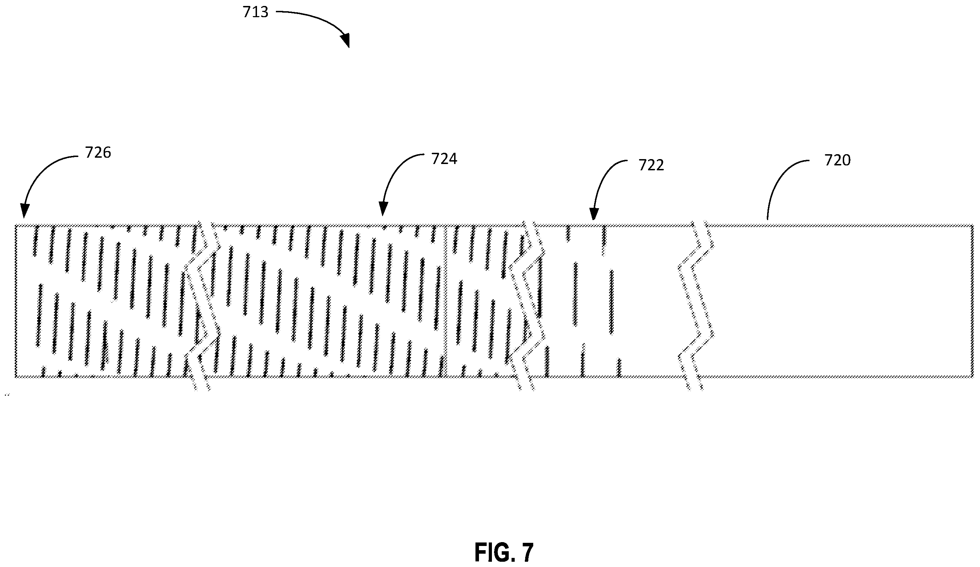

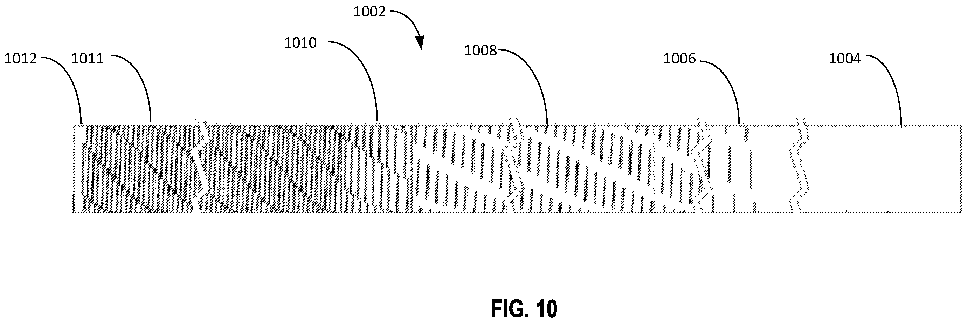

Elongated shaft 102 may include a proximal region and a distal region. The proximal region may be connected to the handle and the distal region may be connected to the deployment assembly. Elongated shaft 102 may include one or more cut hypotubes having a proximal portion, a transition portion cut to have greater flexibility than the proximal portion, and a distal portion cut to have greater flexibility than the transition portion. For example, as illustrated in FIG. 7, elongated shaft 102 may include hypotube 713 which may include transition section 722 which may increase in cut density moving in a proximal to distal direction, constant section 724 which may have a constant cut density that may be more dense that transition section 722, and no-cut section 726, which may be a distal region of hypotube 713 with no cuts. In another example, as illustrated in FIG. 10, elongated shaft 102 may include guidewire hypotube 1002 which may include transition section 1006 which may increase in cut density moving in a proximal to distal direction, low density section 1008 which may have a constant cut density that may be more dense that transition section 1006, transition section 1008 which may increase in cut density moving in a proximal to distal direction and may be more dense than low density section 1008, and high density section 1011 which may have a constant cut density that may be more dense than transition section 1008.

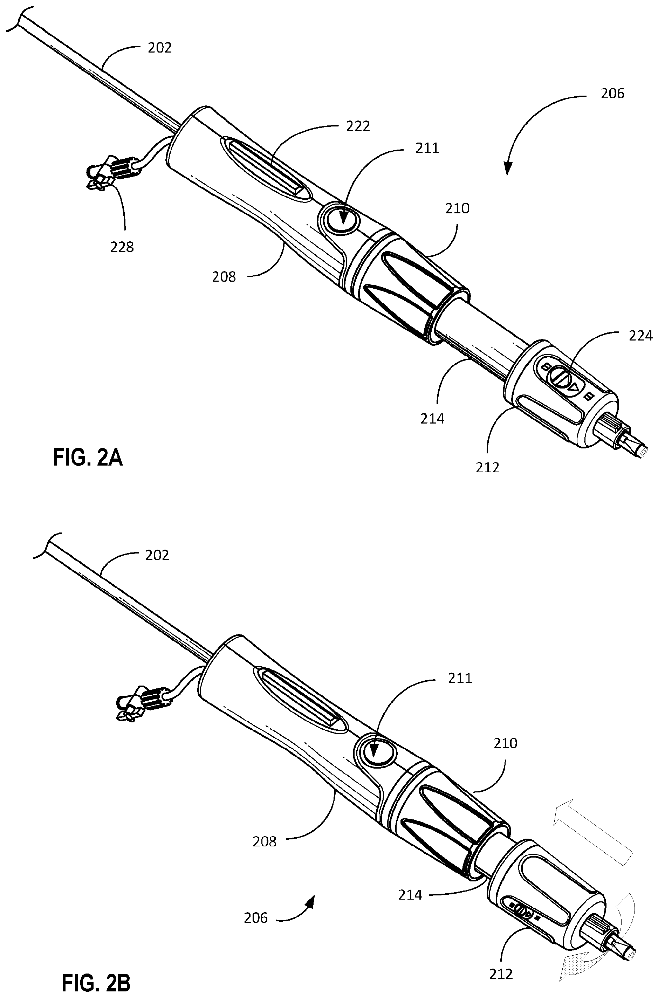

Referring now to FIGS. 2A-2B, perspective views of exemplary handle 206 are illustrated. Handle 206 may be the same as or similar to handle 106 of FIG. 1. As shown in FIGS. 2A-2B, handle 206 may be coupled to elongated shaft 202. Elongated shaft 202 may be the same as or similar to clongated shaft 102 of FIG. 1. Handle body 208 may be the same or similar to handle body 108 of FIG. 1.

Handle 206 may be disposed at a proximal region of elongated shaft 206. Handle 206 may include handle body 208 and positioning actuator 212. Positioning actuator 212 may be designed to rotate relative to handle body 208. Handle 206 may include releasing actuator 206 which may be located on positioning actuator 212 and/or which may be designed to transition between a first position (e.g., a locked position) and a second position (e.g., an unlocked position). When releasing actuator 206 is in the first position, rotation of positioning actuator 212 may be translated to the deployment assembly to cause the deployment assembly to rotate. When releasing actuator 224 is in the second positon, rotation of positioning actuator 212 may cause the distal sleeve of the deployment assembly to move longitudinally relative to handle 206 for expanding and implanting the prosthetic heart valve.

Handle body 208 may be tubular in shape and may be ergonomic in size and shape to facilitate easy handling for the user. For example, handle body may have an hourglass or diablo shape. Handle body 208 may further include indicator 222 which may include a window or slot along which an indicator may traverse. Indicator 222 may be in mechanical communication with deflection actuator 210 and may indicate a degree of activation and/or degree of deflection in elongated shaft 202. Handle body 208 may further include port 228, which may be in fluid communication with an internal channel in handle body 208 and/or one or more channels of elongated shaft 202 and may be used to flush the handle body 208 and/or one or more channels of elongated shaft 202 (e.g., with saline solution).

Handle body 208 may further include unlocking actuator 211 which may be positioned in a proximal region of handle body 208. Unlocking actuator 211 may be a button or other engagable (e.g., depressible) feature that may permit positioning actuator 212 and positioning shaft 214 to rotate and move axially with respect to handle body 208. For example, as shown in FIG. 2B, when unlocking actuator 211 is engaged (e.g., depressed), and therefore unlocked, positioning actuator 212 may be advanced axially in the distal direction while handle body 108 is maintained in place. Advancing positioning actuator 212 distally causes elongated shaft 202 and all internal shafts, wires, cables, and the like, to similarly advance distally, thereby causing the entirety of the deployment assembly to advance distally. Similarly, rotating positioning actuator 212 when unlocking actuator 211 is in the unlocked position (i.e., the depressed position), causes elongated shaft 202 to similarly rotate. When unlocking actuator 211 is not engaged and thus in the locked position and positioning actuator 212 is in a locked position (e.g., releasing actuator 224 is in a locked position) such that positioning actuator 212 is fixed to positioning shaft 214, positioning actuator 212 and positioning shaft 214 may be fixed axially and/or rotationally with respect to handle body 208.

Deflection actuator 210 may include internal threads that may engage threads housed internally within handle body 208. Deflection actuator 210 may be tubular in shape, may be tapered at one end, and may have a series of ridges to facilitate grip by the user. Deflation actuator 210 may have an internal channel sized to receive positioning shaft 214, which may be secured to handle body 208 and positioning actuator 212. As deflection actuator 210 is rotated, deflection actuator 210 may cause internal threading of handle body 208 to move proximally. The internal threading may be mechanically connected to deflection indicator 222 and may further be mechanically connected to a wire or cable connected to the end region of elongated shaft 202 such that retraction of the wire or cable causes elongated shaft 202 to deflect (e.g., bend or arch). The degree to which elongated shaft 202 has deflected may be displayed via deflection indicator 222.

Positioning actuator 212 is similarly tubular in shape and may be tapered on one end. Also like deflector actuator 210, positioning actuator 212 may have a series of ridges to facilitate grip by the user and may also be threaded internally. Positioning actuator 212 may be selectively connected to positioning shaft 214 and may include an internal channel sized to receive a deployment base. The deployment base may be rigidly connected to the guidewire shaft within elongated shaft 202. When releasing actuator 224 is in the unlocked position, the deployment base may selectively interface with the positioning actuator 212 via a threaded interface to cause the deployment base and thus the guidewire shaft to advance distally, thereby causing the distal sleeve and the anchor support of the delivery assembly to advance distally. Positioning actuator 212 may further include releasing actuator 224, which may reversibly fix positioning actuator 212 to positioning shaft 214. Releasing actuator 224 may slidably engage positioning shaft 214 and may transition from a locked position to an unlocked position.

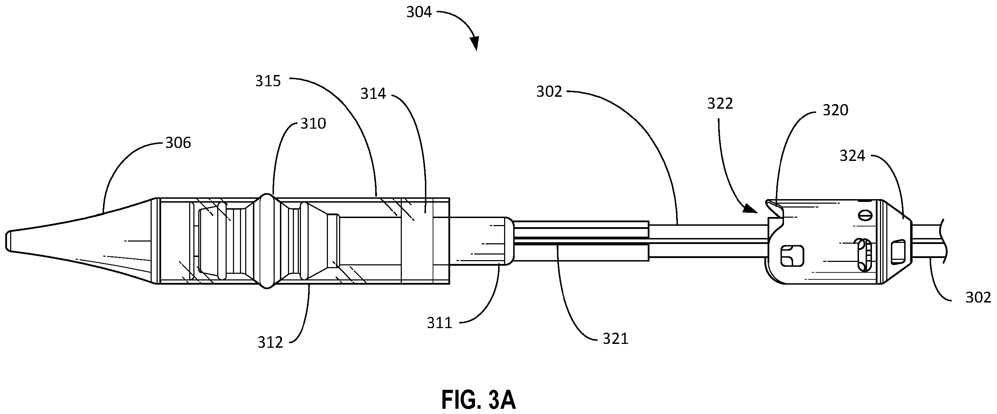

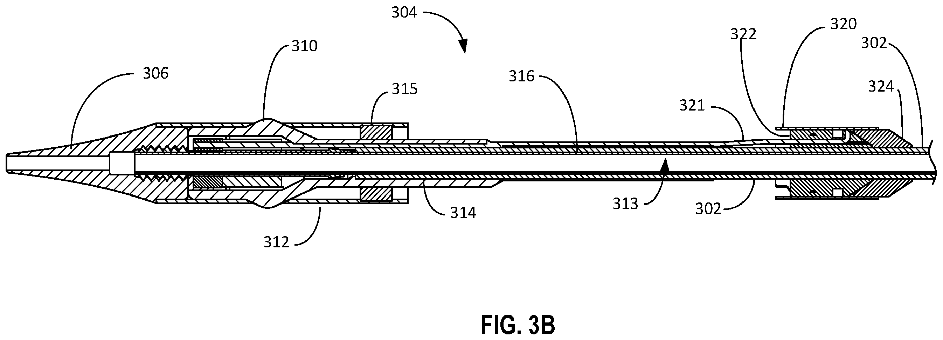

Referring now to FIGS. 3A-3D, perspective and cross-sectional views of exemplary deployment assembly 304 is illustrated. Deployment assembly 304 may be the same as or similar to deployment assembly 104 of FIG. 1. As shown in FIGS. 3A and 3B, deployment assembly 304 may be connected to elongated shaft 302, which may be the same as elongated shaft 102 of FIG. 1. For example, proximal taper 324 may be connected to elongated shaft 302. Proximal taper 324 may be a tapered structure and may be tapered towards the proximal end. Proximal taper 324 may be coupled to proximal sleeve 320. For example, proximal taper 324 may be rigidly coupled to elongated shaft 302 and proximal sleeve 320 may be rigidly connected to proximal taper 324.

At least a portion of elongated shaft 302, such as the torque tube, may be rigidly coupled to and terminate at retraction taper assembly 314. Retraction taper assembly 314 may be designed to support a cardiac implant such as a prosthetic heart valve. For example, retraction taper assembly 314 may include cylindrical seat portion 311 and conical portion 310. Conical portion 310 may have dual conical portions meeting at a diameter larger than cylindrical seat portion 311. Conical portion 310 may be compressible permitting conical portion 310 to compress to a smaller diameter than the non-compressed state.

Deployment assembly 304 may be sized and shaped to be advanced to an implantation site at a native heart valve site with the prosthetic heart valve in a collapsed state. Deployment assembly 304 may include proximal sleeve 320, anchor support 322, which may be positioned within sleeve proximal sleeve 320 during delivery to maintain at least a portion of the prosthetic heart valve in the collapsed state between the proximal sleeve 320 and the anchor support 322, and a lock (e.g., ball or lever) designed to lock proximal sleeve 320 to anchor support 322 during delivery. A handle (e.g., positioned at a proximal region of elongated shaft 302) may be designed to, when actuated, cause the lock (e.g., ball or lever) of deployment assembly 304 to unlock such that anchor support 322 is longitudinally moveable relative to proximal sleeve 320 for expanding and implanting the prosthetic heart valve.

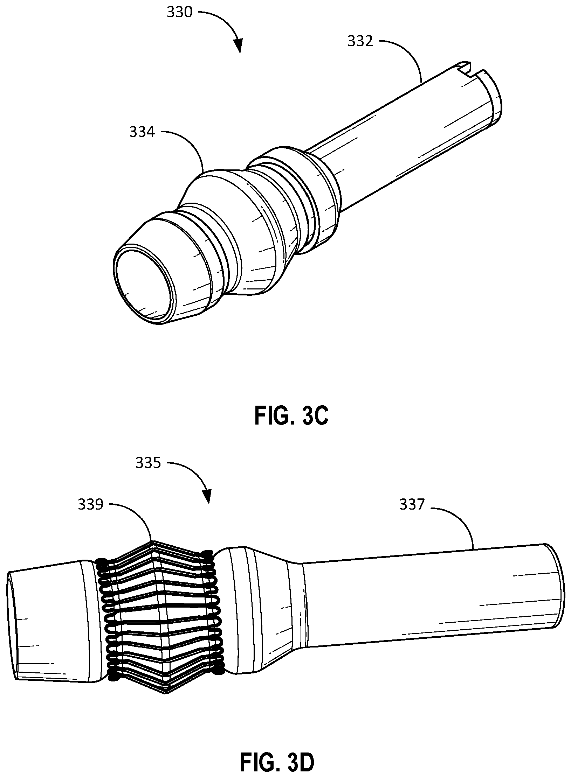

Retraction taper assemblies are illustrated in FIGS. 3C-3D. As shown in FIG. 3C, retraction taper 310 assembly may include tubular portion 332 and conical portion 334, which may have a central channel to receive tubular portion 332 and may be constrained in place by tubular portion 332. It is understood that conical portion 334 and/or tubular portion 332 may be compressible and/or elastic (e.g., foam). Referring now to FIG. 3D, an alternative retraction taper assembly, retraction taper assembly 335, may be similar to retraction taper assembly 330 but may include tubular portion 332 which may have a tubular shape and compressible mesh 339 which may be a metallic mesh (e.g., laser cut Nitinol bulb). Tubular portion 332 may be compressible and/or elastic.

Referring again to FIGS. 3A-3B, guidewire shaft 313 may be positioned within elongate shaft 302 and may extend to and terminate at distal sleeve 312. Distal sleeve 312 may include or may be connected to end cone 306, which may be tapered in the proximal direction. Distal sleeve 312 may have a diameter larger than cylindrical seat portion 311 but smaller than conical portion 310. Accordingly, the open end of distal sleeve 312 may be manipulated to abut conical portion 334. Distal sleeve 312 may also forcefully traverse over conical portion 310, causing conical portion 310 to compress, as shown in FIGS. 3A-3B. It may be preferable for distal sleeve 312 to traverse conical section 310 such that an open end of distal sleeve 312 may retain a distal end of the prosthetic heart valve positioned on cylindrical seat portion 311. Distal sleeve 312 may further include imaging marker 315, which may appear using well known medical imaging. For example, imaging marker 315 may be a ring position on a proximal region of distal sleeve 312. This positioning may be desirable as it may be helpful for an individual deploying the prosthetic heart valve to know when the prosthetic heart valve is exiting an introducer sheath. In one example, imaging marker 315 may be a radiopaque or other well-known image marker.

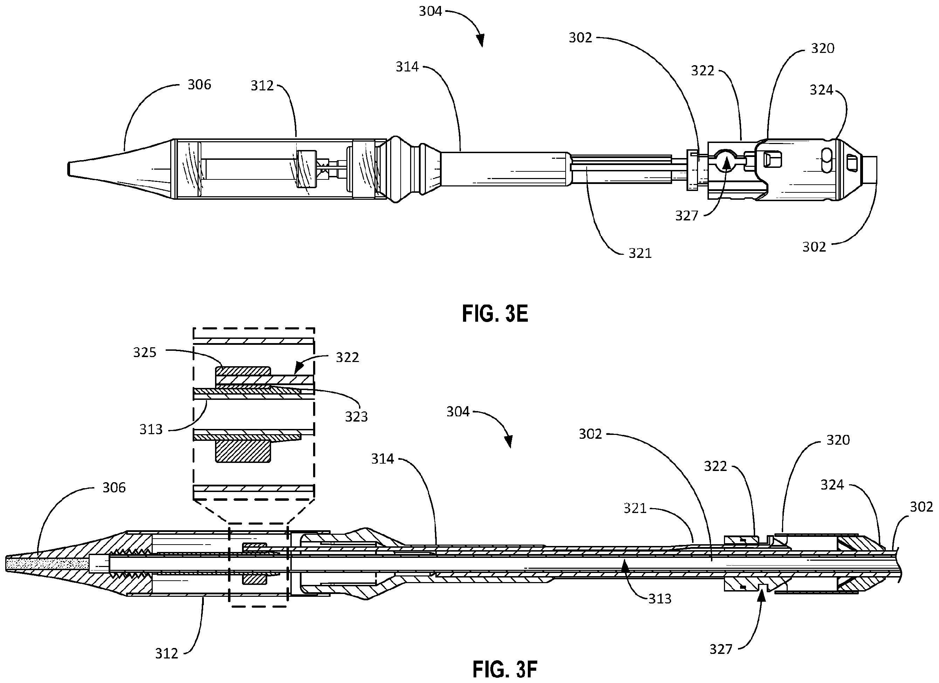

Distal sleeve 312 and/or end cone 306 may include an internal protrusion or catch (e.g., catch 323 of FIG. 3F) that, as distal sleeve 312 moves distally, may engage or interface with anchor connector 321. Anchor connector 321 may be a cable, wire, tube, or the like and may similarly include a protrusion or catch (e.g., catch 325 of FIG. 3G) that engages or interfaces the protrusion or catch near or on distal sleeve 312 and/or end cone 306. It is understood that the protrusion or catch of distal sleeve 312 may be positioned such that distal sleeve 312 may move distally a certain length before the protrusion or catch of distal sleeve 312 engages or interfaces with the protrusion or catch of anchor connector 321.

At a proximal end, anchor connector 321 may connect with anchor support 322, which may be sized to fit within proximal sleeve 320. Alternatively, anchor support may connect to an internal support within and interfacing with anchor support 322. Anchor support 322 may be designed to receive one or more anchors or structural elements of the prosthetic heart valve and maintain the anchors or structural elements of the prosthetic heart valve between anchor support 322 and distal sleeve 320. When anchor connector 321 is moved distally by distal sleeve 312 and/or end cone 306, anchor support 322 may move distally the same amount. As proximal sleeve 320 is rigidity connected to proximal taper 324 and elongated shaft 302, when anchor support 322 is advanced distally, proximal sleeve 320 remains in place. As a result, any anchors or structural elements positioned between anchor support 322 and proximal sleeve 320 may be exposed and released when anchor support 322 is advanced distally from proximal sleeve 320.

In the arrangement illustrated in FIGS. 3A and 3B, deployment assembly 304 may be positioned to retain a cardiac implant such a self-expanding prosthetic heart valve in a collapsed or partially collapsed state. For example, a distal end of the prosthetic heart valve may be positioned within the open distal end of distal sleeve 312 such that the distal end of the prosthetic heart valve may be retained and compressed by distal sleeve 312. Similarly, the proximal end of the prosthetic heart valve may have one or more anchor or structural elements positioned between anchor support 322 and proximal sleeve 320 such that the proximal end of the prosthetic heart valve may be retained and compressed by proximal sleeve 320 and anchor support 322.

Referring now to FIGS. 3E-3F, deployment assembly 304 is illustrated and positioned for release of a prosthetic heart valve. As shown in FIGS. 3E-3F, distal sleeve 312 and end cone 306 may be advanced distally past conical section 310 of retraction taper assembly 314, thereby releasing a distal end of the prosthetic heart valve and permitting at least the distal portion of the prosthetic heart valve to expand to an expanded state. Subsequently, anchor support 322 may be advanced distally by anchor connector 321 as anchor connector 321 is advanced distally by distal sleeve 312 and/or end cone 306. As anchor support 322 is advanced distally, anchor recessions 327 of anchor support 322 may be exposed from distal sleeve 320.

Anchor recessions 327 may be recessions or structure (e.g., protrusions) on anchor support designed to receive an anchor or other structural feature of the proximal end of the prosthetic heart valve. As anchor recessions 327 are exposed from proximal sleeve 320, the proximal end of the prosthetic heart valve is permitted to expand to an expanded state. It is understood that exposure of the anchor recessions 327 from distal sleeve 320 may fully release the prosthetic heart valve from deployment assembly 304.

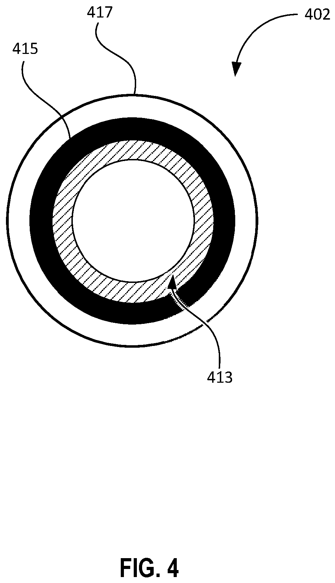

Referring now to FIG. 4, a cross-sectional view of an exemplary elongated shaft is illustrated. Elongated shaft 402 may be the same or similar to elongated shaft 102 of FIG. 1. Elongated shaft 402 may include three distinct shafts including deflection shaft 417, torque shaft 415, and guidewire shaft 413. It is understood that additional layers may be included in elongated shaft 102. Deflection shaft 417, torque shaft 415, and/or guidewire shaft 413 may move axially and/or rotationally independently. In one example. Deflection shaft 417 and torque shaft 415 may instead be fixed to one another. Deflection shaft 417 may serve as a spine for elongated shaft 402 and may be attached in a distal region to a deflecting wire (not shown) that may extend from the handle to the distal region of deflection shaft 417. As the handle causes the deflecting wire to move axially and proximally towards the handle, deflection shaft 417 may be caused to arch or bend.

Torque shaft 415 may be positioned within deflection shaft 417 and may extend between the positioning shaft of the handle and the retraction taper assembly of the deployment assembly. In this manner, torque shaft 415 may be designed to transfer torque from the positioning shaft to the deployment assembly. Guidewire shaft 413 may be positioned within torque shaft 415 and may extend between the deployment base positioned within the positioning actuator of the handle and the distal sleeve and/or end cone of the deployment assembly.

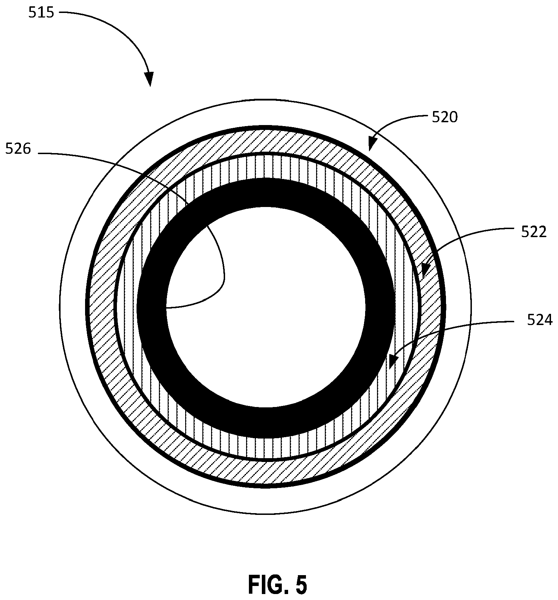

Referring now to FIG. 5, torque shaft 515 may be made of several layers. For example, torque shaft 515 may have hypotube layer 520 that may be a hypotube that is strategically cut to increase in flexibility from a proximal to distal direction. For example, the hypotube may be a laser cut hypotube having a plurality of cut through-holes or slits created using a laser. Polymer layer 522 may be positioned within hypotube layer 520. Polymer layer 522 may include a polymer jacket that may be positioned within the hypotube. The polymer jacket may include stiff polymers (e.g., nylon polymers). Braid layer 524 may be positioned within polymer layer 522. Braid layer 524 may be a metallic braid. It is understood that the polymer jacket may flow into braid layer 524. In one example, braid layer 524 may include a metallic braid that may include stainless steel and Kevlar fiber. The braid may be any other braid including metal, allows and/or composite materials. Liner layer 526 may be an innermost layer positioned within braid layer 524. Liner layer 526 may be a liner including a fluoropolymer such as polytetrafluoroethylene (PTFE) or other similar material. It is understood that polymer layer 522, braid layer 524 and liner layer 526 may form a composite shaft. It is further understood that torque shaft 515 may have additional or fewer layers than those shown in FIG. 5.

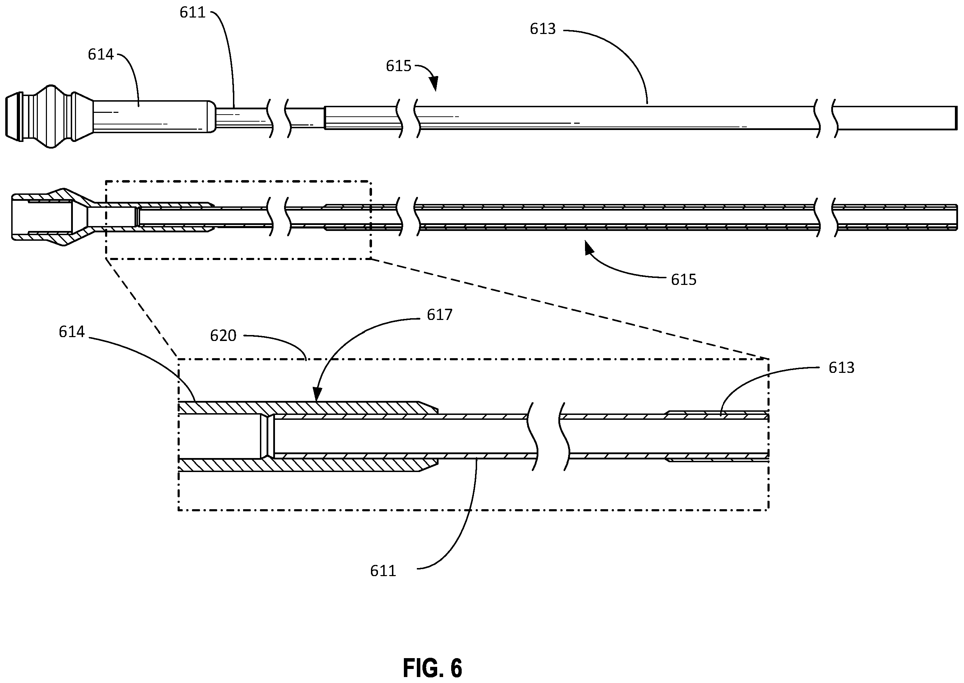

Referring now to FIG. 6, side and cross-sectional view of torque shaft 615 are illustrated. As shown in FIG. 6, torque shaft 615 may connect to retraction taper assembly 614 at a distal end of torque shaft 615. Retraction trapper assembly 614 may be the same as or similar to retraction taper assembly 314 of FIG. 3A or retraction taper assembly 330 of FIG. 3C. Torque shaft 615 at a proximal region may include composite shaft 611 (e.g., poloymer layer 522, braid layer 524, and liner layer 526 of FIG. 5) as well as hypotube 613 cut to facilitate increased flexibility from the proximal to distal direction. Near the distal region of torque shaft 615, hypotube 613 may terminate and only composite shaft 611 of torque shaft 615 may continue to retraction taper assembly 614. This may facilitate a reduced diameter of torque shaft 615 near the distal end of torque shaft 615. As shown in view 620, composite shaft 611 may extend within retraction taper assembly 614 and retraction taper assembly may include composite shaft receiving channel 617 to secure composite shaft 611.
Referring now to FIG. 7, torque hypotube 713 is illustrated. Torque hypotube 713 may be the outmost layer of torque shaft 515 of FIG. 5. Torque hypotube 713 may be metallic, composite, and/or plastic, for example, and may be tubular in shape. In one example, torque hypotube 713 may be stainless steel and may have a cut design from laser cutting. As shown in FIG. 7, the cuts made into torque hypotube 713 may be perpendicular to the longitudinal axis of the torque tube and increase in density (e.g., number of cuts per given area) moving from the proximal to distal direction. Proximal region 720 of hypotube 713 may not have any cuts.

Hypotube 713 may have sections of consistent repeating pattern of cuts. For example, hypotube 713 may include transition section 722 which may increase in cut density moving in a proximal to distal direction, constant section 724 which may have a constant cut density that may be more dense that transition section 722, and no-cut section 726, which may be a distal region of hypotube 713 with no cuts. In transition section 722, cuts may progressively transition with distance between each cut gradually decreasing from the proximal to distal direction. Additionally, the cuts may be designed to spiral about hypotube 713.

With the cut arrangement illustrated in FIG. 7, torque tube 713 may increase in flexibility from the proximal to distal direction with the increased density of cuts from the proximal to distal end. While it is understood that the dimensions and design of the cuts may be vary, in one non-limiting example, the width of each cut may be 5.08 mm (0.20 inches), the pitch may vary between 0.203 and 1.016 mm (0.008 and 0.04 inches), the cut length may vary between 15.24 to 17.78 mm (0.6 to 0.7 inches), and the cut angle may vary between 62 and 74 degrees. It is further understood that the arrangement and length of the various cut sections of torque hypotube 713 may be the same.

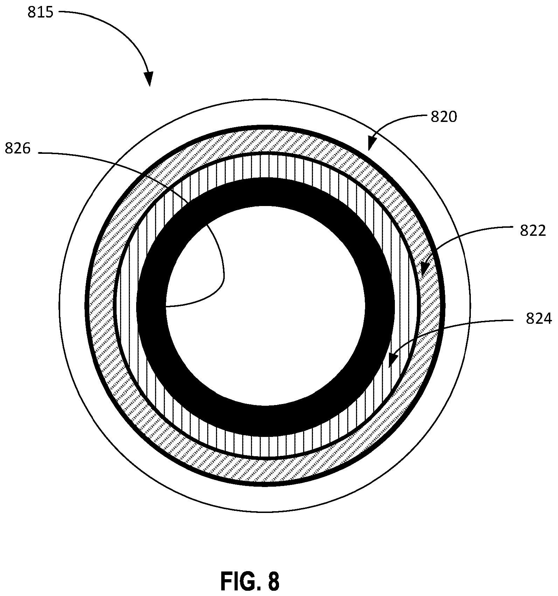

Referring now to FIG. 8, guidewire shaft 815 may be made of several layers. For example, guidewire shaft 815 may have hypotube layer 820 that may be a hypotube that is strategically cut to become more flexible from a proximal to distal direction. For example, the hypotube may be a laser cut hypotube. Polymer layer 822 may be positioned within hyptoube layer 820. Polymer layer 822 may include a polymer jacket that may be positioned within the hypotube. The polymer jacket may include stiff polymers (e.g., nylon polymers). Braid layer 824 may be positioned within polymer layer 822. Braid layer 824 may be a metallic braid. It is understood that the polymer jacket may flow into braid layer 824. In one example, braid layer 824 may include a metallic braid that may include stainless steel and Kevlar fiber. The braid may be any other braid including metal, allows and/or composite materials. Liner layer 826 may be an innermost layer positioned within braid layer 824. Liner layer may be a liner including polytetrafluoroethylene (PTFE) or other similar material. It is understood that polymer layer 822, braid layer 824 and liner layer 826 may form a composite shaft.

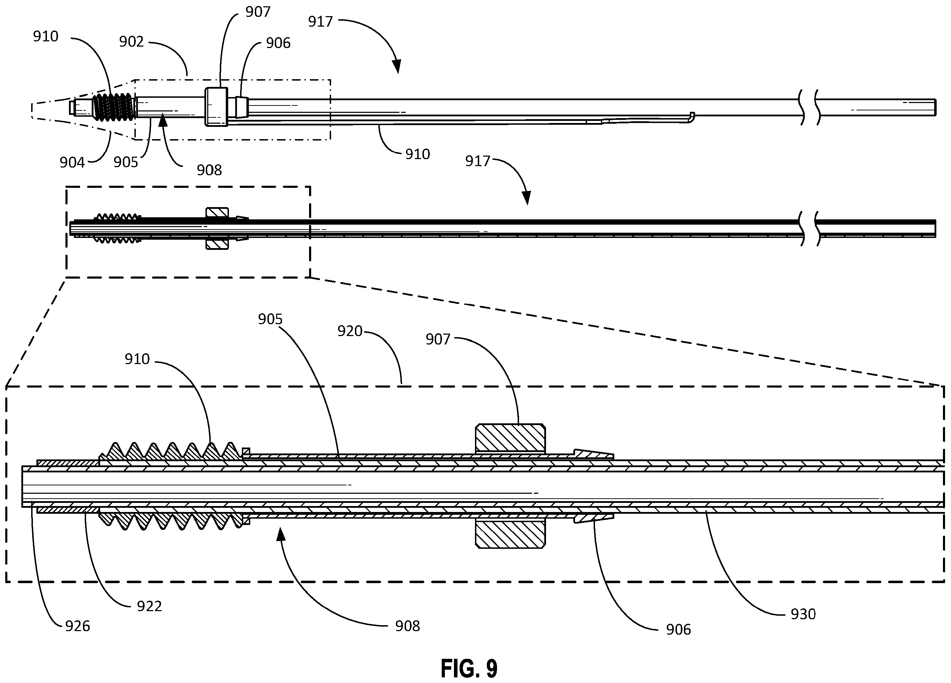

Referring now to FIG. 9, side and cross-sectional view of guidewire shaft 917 is illustrated. As shown in FIG. 9, guidewire shaft 917 may connect to distal sleeve 902 and end cone 904, which may be the same or similar to distal sleeve 312 and end cone 306 of FIG. 3A, respectfully. As shown in view 920, guidewire shaft 917 may include composite shaft 926 (e.g., poloymer layer 822, braid layer 824, and liner layer 826 of FIG. 8) as well as hypotube 930 cut to provide increased flexibility from the proximal to distal direction. Near the distal region of guidewire shaft 917, hypotube 930 and/or composite shaft 926 may connect to catch assembly 908, which may include catch body 905, which may be tubular in shape, catch 906 at a proximal end of catch body 905, which may be a cylindrical catch having a diameter larger than catch body 905 but smaller than distal sleeve 902, and threads 910 which may be a threaded portion at the distal region of catch body 905. Threads 910 may engage with distal cone and may facilitate a rigid engagement between catch assembly 908 and end cone 904 and distal sleeve 902.

Catch assembly 908 may further include slide 907, which may be tubular in shape and slide on catch body 905. Slide 908 may be connected to anchor connector 910, which may be a wire or elongated structure that extends from catch assembly 908 to the anchor support (not shown). Catch 906 may have an outer diameter that is larger than an inner diameter of slide 907 and catch 906 may engage slide 907 as guidewire shaft 917 is advanced distally causing slide 907 to similar move distally, thereby pulling anchor connector 910 and thus anchor support distally. As shown in view 920, hypotube 930 may extend distally beyond catch assembly 908. Extended portion 922 of hypotube 930 may be crimped and/or welded. For example, catch assembly 908 may be metallic and hypotube 930 may be welded to catch assembly 908 such that guidewire shaft 917 is rigidly connected to catch assembly 908. Composite shaft 926 may also extend beyond catch assembly 908. For example, composite shaft 926 and hypotube 930 may terminate in end cone 904. In one example, catch assembly 908 may be stainless steel, though it is understood that catch assembly 908 may be any other material.

Referring now to FIG. 10, guidewire hypotube 1002 is illustrated. Guidewire hypotube 1002 may be the outmost layer of guidewire shaft 815 of FIG. 8. Guidewire hypotube 1002 may be metallic, composite, and/or plastic, for example, and may be tubular in shape. In one example, guidewire hypotube 1002 may be stainless steel and may have a cut design from laser cutting. As shown in FIG. 10, the cuts made into torque hypotube 1002 may be perpendicular to the longitudinal axis of the torque tube and increase in density (e.g., number of cuts per given area) moving from the proximal to distal direction. Proximal region 1004 of hypotube 1002 may not have any cuts. Further, distal region 1012 may not have any cuts. For example, distal region 1012 of hyptube 1002 may be crimped or welded. It is understood that hypotube 1002 may have a greater number of cuts than hypotube 713 of FIG. 7 and/or may exhibit greater flexibility. It is further understood that hypotube 1002 may be longer than hypotube 713 of FIG. 7.

Guidewire hypotube 1002 may have sections of consistent repeating pattern of cuts. For example, guidewire hypotube 1002 may include transition section 1006 which may increase in cut density moving in a proximal to distal direction, low density section 1008 which may have a constant cut density that may be more dense that transition section 1006, transition section 1008 which may increase in cut density moving in a proximal to distal direction and may be more dense than low density section 1008, and high density section 1011 which may have a constant cut density that may be more dense than transition section 1008. Between each constant section, cuts may progressively transition in distance, with the distance between cuts gradually decreasing from the proximal to distal direction. Additionally, the cuts may be designed to spiral about guidewire hypotube 1002.
With the cut arrangement illustrated in FIG. 10, guidewire tube 1002 may increase in flexibility from the proximal to distal direction with the increased density of cuts from the proximal to distal end. While it is understood that the dimensions and design of the cuts may be vary, in one example, the width of each cut may be 5.08 mm (0.20 inches), the pitch may vary between 0.127 and 1.016 mm (0.005 and 0.04 inches), the cut length may vary between 0.6858 to 0.9398 mm (0.0270 to 0.0370 inches), and the cut angle may vary between 45 and 62 degrees. It is further understood that the arrangement and length of the various cut sections of hypotube 1002 may be the same.

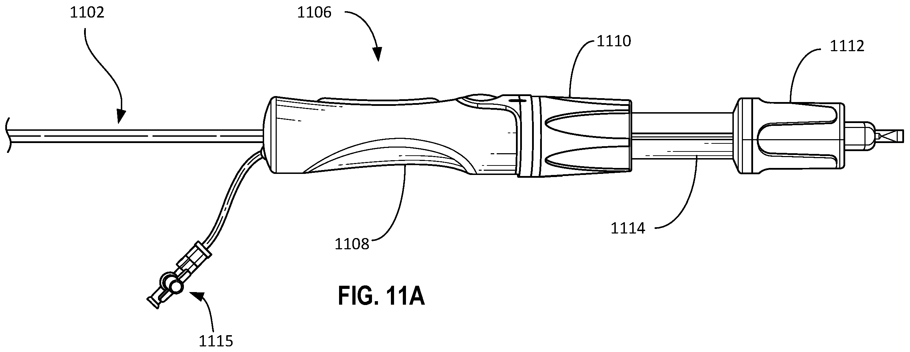

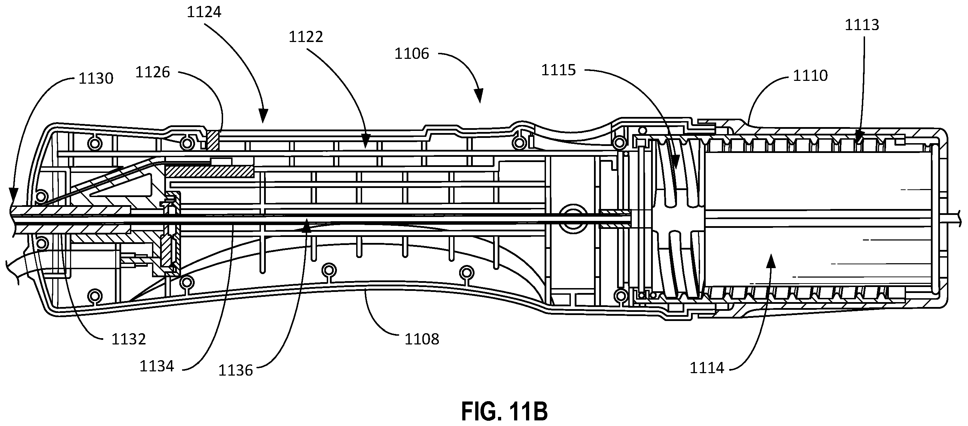

Referring now to FIGS. 11A and 11B, a side and cross-sectional view of handle 1106 is illustrated. Handle 1106 may be the same as or similar to handle 106 of FIG. 1. For example, handle 1106 may include handle body 1108, which may be connected to deflection actuator 1110, positioning shaft 1114, and positioning actuator 1112. Handle body 1108, deflection actuator 1110, positioning shaft 1114, and positioning actuator 1112 may be the same or similar to handle body 108, deflection actuator 110, positioning shaft 114, and positioning actuator 112 of FIG. 1. Handle 1106 may receive elongated shaft 1102, which may be the same or similar to elongated shaft 102 of FIG. 1. Handle 1106 may further include port 1115 which may be connected to one or more interior channels (e.g., elongated shaft 1102) and may be used to flush the one or more interior channels (e.g., using a saline solution).

Referring now to FIG. 11B, a cross-sectional view of handle 1106 is illustrated. Handle body 1108 may be a tubular shell and may be split into halves. Handle body 1108 may have an open end on the proximal region of handle body 1108. Drum support 1114 may be positioned within the open end on the proximal end of handle body. Drum support 1114 may be cylindrical in shape and may smaller in diameter than the open end of handle body 1108. Drum support 1114 may be secured to handle body such that drum support is suspended in the middle of the open proximal end of handle body 1108 such that a gap exists between an outer diameter of drum support 1114 and handle body 1108. Drum support 1114 may extend proximally beyond handle body 1108.

The open proximal end of handle 1108 may be sized to receive deflection actuator 1110. Deflection actuator 1110may be cylindrical in shape and may include thread 1113 on the interior of deflection actuator 1110. Thread 1113 may run all or most of the length of deflection actuator 1110 and may extend distally beyond deflection actuator 1110 such that a portion of thread 1113 enters the open end of handle body 1108 and is secured to a distal end of drum support 1114 in a manner that restricts movement axially but permits rotational movement of deflection actuator 1110. Threaded slider 1115 may be supported by and may slide on drum support 1114. Threaded slider 1115 may be cylindrical and may have threading on its outer surface that is designed to engage with the threading of thread 1113 of deflection actuator 1110. As deflection actuator 1110 is rotated, deployment thread 1113 is similarly rotated causing threaded slider to either move distally or proximally, depending on the direction of rotation, along drum support 1114.

Threaded slider 1115 may be connected to deflector wire 1122 which may be any wire, cable, or the like, and may be connected to a distal region of deflection shaft 1130. Deflector wire 1122 may be guided to an upper portion of handle body 1108 and engaged with indicator 1126. Indicator 1126 may be a protrusion of any other visual indicator that may slide along indicator window 1124 as threaded slider 1115 moves distally or proximally. For example, as deflection actuator 1110 rotates, threaded slider 1115 may be caused to move proximally, pulling deflector wire 1122 proximally, thereby causing indicator 1126 to move proximally and slide along indicator window 1124. Indicator window 1124 may indicate to the user a degree or amount of deflection being applied to deflection shaft 1130. Deflection shaft 1130 may terminate at in a distal region of handle body 1108 and deflector wire 1122 may be introduced into deflection shaft 1130 within handle body 1108. In one example, deflector wire 1122 may be positioned between deflection shaft 1130 and torque shaft 1134. As shown in FIG. 11B, support drum 1114 may be tubular and hollow and torque shaft 1134 and guidewire shaft 1136 may traverse an interior of support drum 1114 and extend proximally beyond drum support 1114, handle body 1108, and deflection actuator 1110.

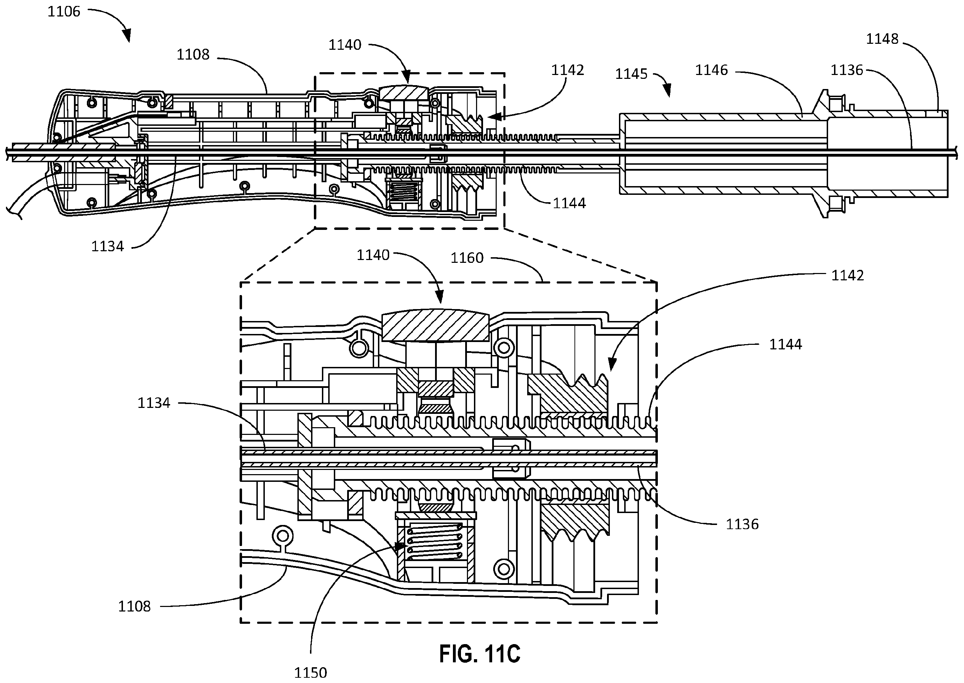

Referring now to FIG. 11C, a cross-sectional view of handle 1106 engaged with positioning shaft 1145 is illustrated. Positioning shaft 1145 may be the same as or similar to positioning shaft 114 of FIG. 1. Positioning shaft 1145 may include threaded shaft 1144, main shaft 1145, and positioning support 1148. Threaded shaft 1144 may be generally tubular in shape with an internal channel and an outer surface having threading or ridges extending along a substantial portion of the outer surface of the shaft. Main shaft 1145 may be tubular in shape and have an internal channel that connects with the internal channel of threaded shaft 1144. Positioning support 1148 may have the same or similar diameter as main shaft 1146 and may have an internal shaft that is similarly sized as the internal channel of main shaft 1145. Threaded shaft 1144, main shaft 1145, and positioning support 1148 may be an integral piece formed from the same material (e.g., plastic), or may be distinct pieces that are connected together.

View 1160 illustrates components within handle body 1108, specifically depicting unlocking actuator 1140. Unlocking actuator 1140 may be the same as or similar to unlocking actuator 211 in FIGS. 2A-2B. Unlocking actuator 1140 may engage with threaded shaft 1144 in an undepressed position to lock threaded shaft 1144 axially and rotationally and may be depressible to permit axial and rotational movement of threaded shaft 1144. Threaded shaft 1144 may be rigidly connected to torque shaft 1134 such that rotational and axial movement of threaded shaft 1144 may be translated to torque shaft 1134. Torque shaft 1134 may terminate within or at threaded shaft 1144. It is understood that while torque shaft 1134 may terminate at or within threaded shaft 1144, guidewire shaft 1136 may extend beyond threaded shaft 1144, main shaft 1146 and into positioning support 1148.

As shown in FIG. 11C, threaded shaft 1144 may traverse rotation limiter 1142. Rotation limiter 1142 may be cylindrical and may have a threaded outer surface and an interior channel having right angles. The threaded outer surface may be positioned within and engage the support drum (not shown). Threaded shaft 1144 may have right angles designed to confirm to an engage the right angles of rotation limiter 1142 such that when threaded shaft 1144 rotates, so too does rotation limiter 1142. Unlocking actuator 1140 may rest on spring assembly 1150 which may be supported and/or coupled to handle body 1108. Spring assembly 1150 may include a spring and a bar positioned at the top of the spring. It is understood that, when unlocking actuator 1140 is depressed, spring assembly 1150 is compressed, threaded shaft and/or main shaft 1145 may be permitted to traverse unlocking actuator 1140 and enter handle body 108 further and may be permitted to rotate with respect unlocking actuator 1140 and handle body 1108.

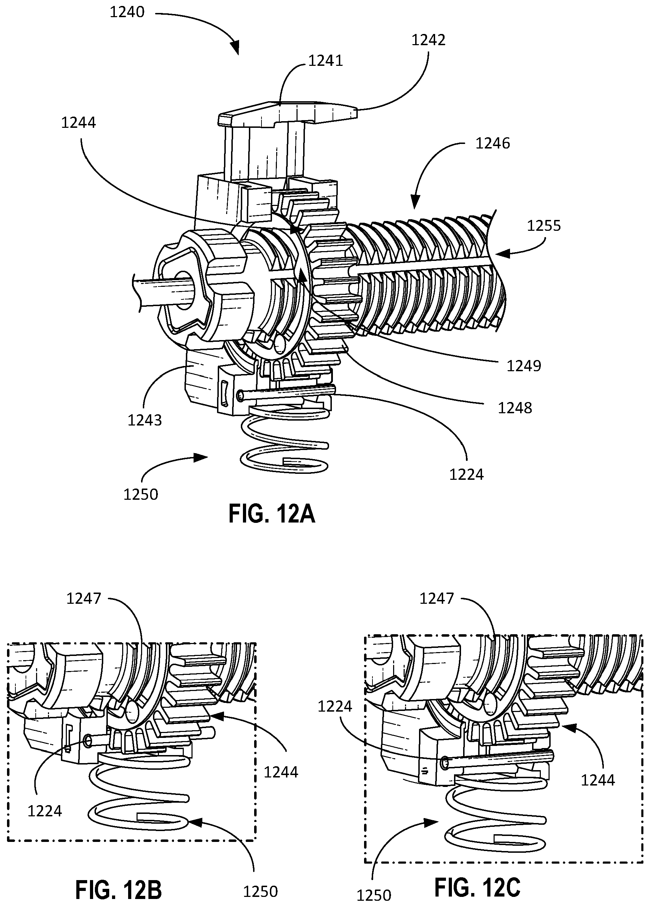

Referring now to FIGS. 12A-12C, perspective cross-sectional views of unlocking actuator 1240 are illustrated. Unlocking actuator 1240 may be the same as or similar to unlocking actuator 211 of FIGS. 2A-2B. As shown in FIGS. 12A-12C, unlocking actuator 1240 may include unlocking body 1242 which may include unlocking button 1241 and wheel housing 1243. Unlocking button 1241 may be a depressible protrusion that may be rigidly connected to wheel housing 1243. Wheel housing 1243 may be generally rectangular and may have a center channel for receiving threaded shaft 1246, which may be the same as threaded shaft 1144 of FIGS. 11C. Wheel housing 1243 may further include a recessed circular channel sized to house ridged wheel 1244 such that ridged wheel 1244 may rotate within wheel housing 1243. Ridged wheel 1244 may be ring-shaped and may have ridges 1248 extending from its exterior surface. The ridges may be linear in shape and extend the length of ridged wheel 1244. Ridged wheel 1244 may further include angular protrusions 1249 extending inwardly. Angular protrusions 1249 may extend the width of ridged wheel 1244 and may extend into thread recessions 1255. Accordingly, rotation of threaded shaft 1246 will cause rotation of ridged wheel 1244.

Unlocking actuator 1240 may further include spring assembly 1250 at the bottom of wheel housing 1243 and bar 1224. Bar 1224 may be connected to bottom portion of wheel housing 123 and may be oriented such that ridges 1248 may receive bar 1224. Spring 1250 may include a spring, which may be a coiled spring. Spring 1250 may interface with wheel housing 1243 on a bottom end of wheel housing 1243 such that downward movement of wheel housing 1243 compresses spring 1250. The recessed portion of wheel housing sized such that wheel housing 1243 may move up and down while ridged wheel 1244 remains at the same height.

Unlocking actuator 1240 may prevent rotation of threaded shaft 1247 in the locked position, illustrated in FIG. 12B. Specifically in the locked position, which may be the default position, spring 1250 may assume a non-compressed state, pushing bar 1224 into a ridge of ridged wheel 1244. With bar 1224 residing in the ridge of ridged wheel 1244, ridged wheel 1244 will be prevented from rotating and thus threaded shaft 1247 will be prevented from rotating. As shown in FIG. 12C, causing unlocking actuator 1240 to move downward will cause spring 1250 of spring assembly 1250 to compress, thereby removing bar 1224 from any ridge of ridged wheel 1244. For example, a user may press down on unlocking button 1241 to compress spring 1250. In the unlocked position shown in FIG. 12C, which may be caused by depressing unlocking actuator downwards, ridged wheel 1244 may be permitted to rotate, thereby permitting threaded shaft 1247 to rotate. Once unlocking actuator is released, spring 1250 will expand towards a neutral position and once again assume the locked position shown in FIG. 12B.

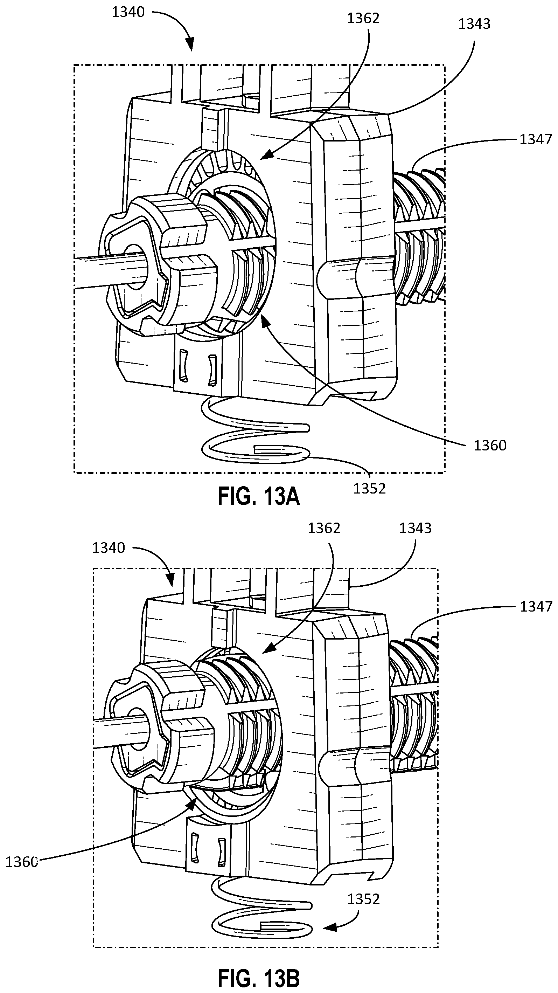

Referring now to FIGS. 13A-13B, unlocking actuator 1340 is illustrated engaged with threaded shaft 1347. Unlocking actuator 1340 may be the same as or similar to unlocking actuator 1240 of FIGS. 12A-C and threaded shaft 1347 may be the same or similar to threaded shaft 1247 of FIGS. 12A-C . As shown in FIGS. 13A-13B, unlocking actuator 1340 may further include thread 1360, which may be position in or near central channel 1362 of wheel housing 1342 which may be the same recessed channel in which the ridged wheel (not shown) rests. Thread 1360 may be sized such that in the locked position with spring 1352 in an expanded state (e.g., the locked position illustrated in FIG. 12B), thread 1360 may interface with one or more threads of threaded shaft 1347. In the locked position, thread 1360 of unlocking actuator 1340 will prevent threaded shaft 1347 from moving axially, whether distally or proximally. As show in FIG. 13B, unlocking actuator 1340 may be transitioned to an unlocked position by compressing spring 1352 (e.g., the unlocked position illustrated in FIG. 12C), thereby moving wheel housing 1343 and thus thread 1360 downward. In the unlocked position shown in FIG. 13B, threaded shaft 1347 may move axially, proximally and distally, without interfacing thread 1360.

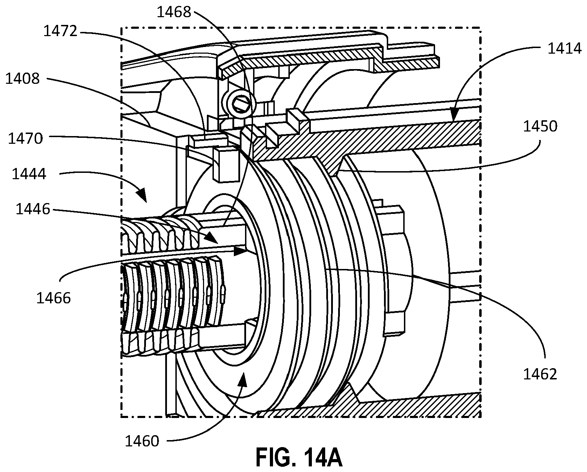

Referring now to FIGS. 14A-14C, support drum 1414 may be secured to handle body 1408. Support drum 1414 may be the same as or similar to support drum 1114 and handle body 1408 may be the same as or similar to handle body 1408 of FIG. 11B. Support drum 1414 may be a tubular in shape and may include an interior surface having interior thread 1450 near a distal end of support drum 1414. Support drum 1414 and interior thread 1450 may engage interior threaded ring 1460. Interior threaded ring 1460 may be annular in shape and may have threaded outer surface 1462. Threaded outer surface 1462 may engage thread 1450 of support drum 1414, such that interior threaded ring 1460 may rotate along thread 1450 and within support drum 1414. In this manner, support drum 1414 may guide interior threaded ring 1460 as it rotates within handle body 1408.

An interior surface of interior threaded ring 1460 may include angular protrusions 1466 that may protrude inwardly from interior threaded ring 1460. For example, angular protrusions 1466 may include a right angle and may be sized to enter thread recession 1446 of threaded shaft 1444. Threaded shaft 1446 may be the same or similar to threaded shaft 1246 and thread recession 1446 may be the same or similar to thread recession 1250 of FIG. 12A. Threaded shaft 1444 may include at a proximal end guides 1468 that may extend outward from threaded shaft 1444 and may maintain thread recession 1446 beyond the more distally shown threads, as shown in FIG. 14A. Threaded shaft 1444 may extend through interior threaded ring 1460 such that threaded shaft 1444 may receive in thread recession 1446 angular protrusions 1466. As threaded shaft 1444 rotates, angular protrusions 1446 cause interior threaded ring 1460 to similarly rotate.

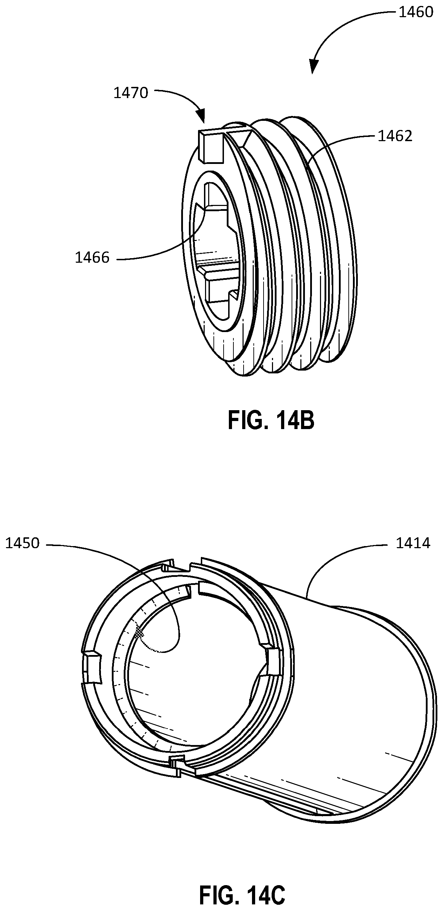

Referring now to FIG. 14B, interior threaded ring 1460 is illustrated. As shown in FIG. 14B, interior threaded ring 1460 may include threaded outer surface 1462 and an interior surface having angular protrusions 1466. Threaded outer surface 1462 may also include stopper protrusion 1470 which may be positioned at the beginning of threaded outer surface 1462 such that stopper protrusions. Stopper protrusion 1470, illustrated in FIGS. 14A-14B, may be designed to engage stopper protrusion 1472 on handle body 1408, shown in FIG. 14A, such that interior threaded ring 1460 may only rotate clockwise and counter clockwise a set degree of rotation before being stopped by handle body 1408 and prevented from further rotation. For example, handle body 1408 may prevent interior threaded ring 1460 and thus threaded shaft 1444 from rotating more than 360 degrees. It is understood that any other amount of rotation may be achieved (e.g., 90, 180, 270, 720, etc.) by changing the number and spacing of threads and/or the locations of the stopper protrusion 1470. It is further understood that more than one stopper protrusion may be included on threaded ring 1460.

Referring now to FIG. 14C, support drum 1414 is illustrated. As shown in FIG. 14C, support drum 1414 may be tubular in shape with an interior surface and an exterior surface. The exterior surface and the exterior surface may be generally smooth. The interior surface may include thread 1450 that may guide the interior threaded ring along the interior of support drum 1414. In one example, thread 1450 and/or the interior surface of support drum 1414 may include one or more stopper protrusions for preventing the interior threaded ring from rotation at a certain point along support drum 1414. Alternatively, the stopper protrusions may be positioned on the handle body.

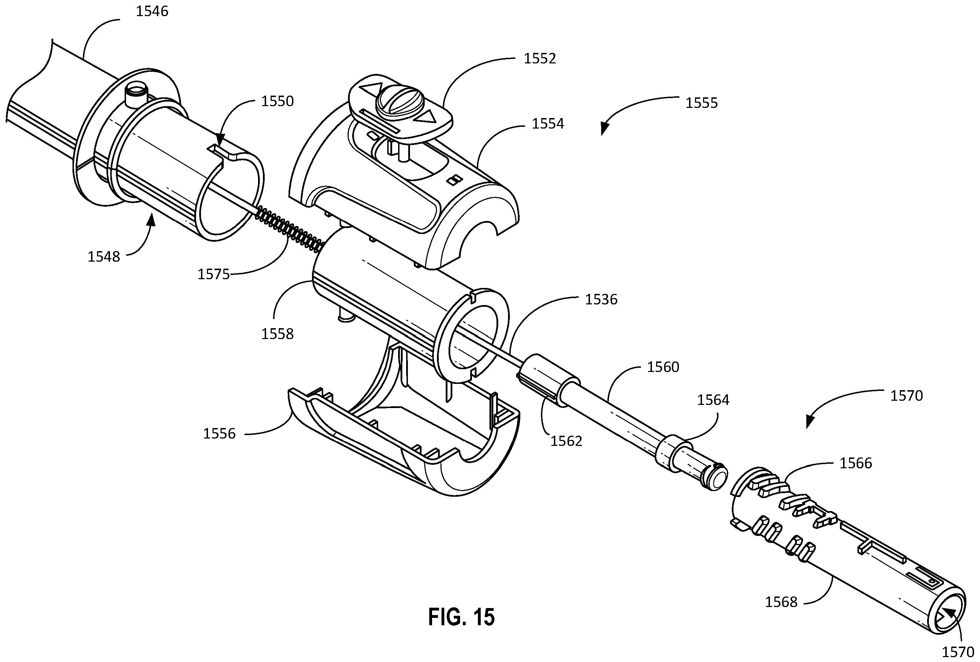

Referring now to FIG. 15, an exploded perspective view of positioning actuator 1555 is illustrated. Positioning actuator 1555 may be the same as or similar to positioning actuator 1112 of FIG. 11A. Positioning actuator 1555 may include positioning actuator housings 1554 and 1556, which may form two halves of positioning actuator 1555, and together may be tubular in shape. Positioning actuator 1555 may further include actuator base 1558, which may be rigidly secured between positioning actuator housings 1554 and 1556. Actuator base 1558 may be threaded on an interior surface. Positioning actuator housings 1554 and 1556 may be secured to positioning support 1548 which may be coupled to or otherwise extend from positioning shaft 1546. Positioning support 1548 and positioning shaft 1546 may be the same as or similar to positioning support 1148 and positioning shaft 1146 of FIG. 11C, respectively. Positioning actuator housing 1554 may include releasing actuator 1552 which may releasably fix positioning actuator 1555 to positioning support 1548. As shown in FIG. 15, releasing actuator 1552 may be a button or similar protrusion that may slide along positioning actuator housing 1554 and may selectively engage receiving area 1550 of positioning support 1548. Receiving area 1550 may be a rectangular void, slit, catch, or the like on positioning support 1548 for interfacing with releasing actuator 1552. Releasing actuator 1552 may also or alternatively be positioned on positioning actuator housing 1556.

Deployment base 1570 may be positioned within actuator base 1558 and may include threaded shaft 1568 and interior shaft 1560. Threaded shaft 1568 may be tubular in shape and may have an internal channel in which interior shaft 1560 may be positioned. Threaded shaft 1568 may include several threads 1566 arranged on an outer surface of threaded shaft 1568 to interface with interior threads of actuator base 1558 such that rotation of actuator base 1558 causes axial movement deployment base 1570. The pattern of threads 1566 may be selected such that rotation of actuator base 1558 advances or withdraws threaded shaft 1568 axially with rotation of actuator base 1558. Interior shaft 1560 may be rigidly connected to guide wire shaft 1536, which may be the same or similar to guidewire shaft 917 of FIG. 9. Accordingly, axial movement of interior shaft 1560 may cause axial movement of guidewire shaft 1536.

Interior shaft 1560 may be tubular in shape and may include rotational fixation protrusion 1562, which may connect to or otherwise interface with threaded shaft 1568 such that rotational and/or axial movement of threaded shaft 1568 is translated to interior shaft 1560. Rotational fixation protrusion 1562 may be a rectangular protrusion or any catch or interfacing structure. Interior shaft 1560 may further include proximal protrusion 1564 which may be cylindrical in shape and may have a larger diameter than the body of interior shaft 1560. Proximal protrusion 1564 may interface with proximal region 1570 of threaded shaft 1568 to secure interior shaft 1560 to threaded shaft 1568 to prevent interior shaft 1560 from advancing axially beyond a certain point. Spring 1575 may be positioned around guidewire shaft 1536 and may interface with a distal end of the positioning shaft and the distal end of interior shaft 1560. As interior shaft 1560 is advanced, the spring may be compressed against the distal end of the positioning shaft and as interior shaft 1560 is advanced towards the distal end of the positioning shaft, the spring force from spring 1575 may increase, causing an increasing force against interior shaft 1560 in the proximal direction. Spring 1575 may cause interior shaft 1562 to return to its proximal most position axial force is not being applied to interior shaft 1560 in the distal direction.

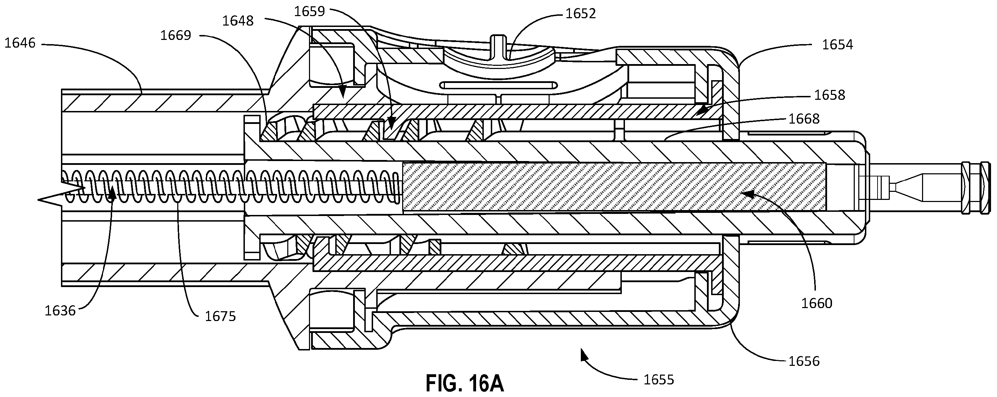

Referring now to FIGS. 16A-16B, cross-sectional views of interior shaft 1660, threaded shaft 1668, actuator base 1658, positioning actuator housings 1654 and 1656, positioning support 1650, positioning shaft 1648, guidewire shaft 1636, and spring 1675 are illustrated. It is understood that interior shaft 1660, threaded shaft 1668, actuator base 1658, positioning actuator housings 1654 and 1656, positioning support 1648, positioning shaft 1646, guidewire shaft 1636, and spring 1675 may be the same as or similar to interior shaft 1560, threaded shaft 1568, actuator base 1558, positioning actuator housings 1554 and 1556, positioning support 1550, positioning shaft 1548, guidewire shaft 1536, and spring 1575 of FIG. 15.

Positioning actuator 1655, may include positioning actuator housings 1654 and 1656 and may further include releasing actuator 1652, which may releasably fix positioning actuator 1655 to positioning support 1648, which may be connected to or extend from positioning shaft 1646. Positioning actuator housings 1654 and 1656 may be secured to positioning base 1658 such that positioning support 1648 is positioned between positioning actuator housings 1654 and 1656 and positioning base 1658. Positioning base 1658 may include internal thread 1659. While internal thread 1659 is illustrated near a distal region of positioning base 1658, it is understood that internal thread 1659 may be positioned near any other portion of positioning base 1658.

Internal thread 1659 of positioning base 1658 may interface with threads 1669 of threaded shaft 1668 such that as positioning base 1659 is rotated, threaded shaft 1668 is caused to move axially, either distally or proximally. Interior shaft 1660 may be secured to threaded shaft 1668 such that axial movement of threaded shaft 1668 is translated to interior shaft 1660. Interior shaft 1660 may be secured to guidewire shaft 1636 (e.g., to a distal region of interior shaft 1660). Spring 1675 may be positioned around guidewire shaft 1636 and may resist axial movement of interior shaft 1660in the distal direction.

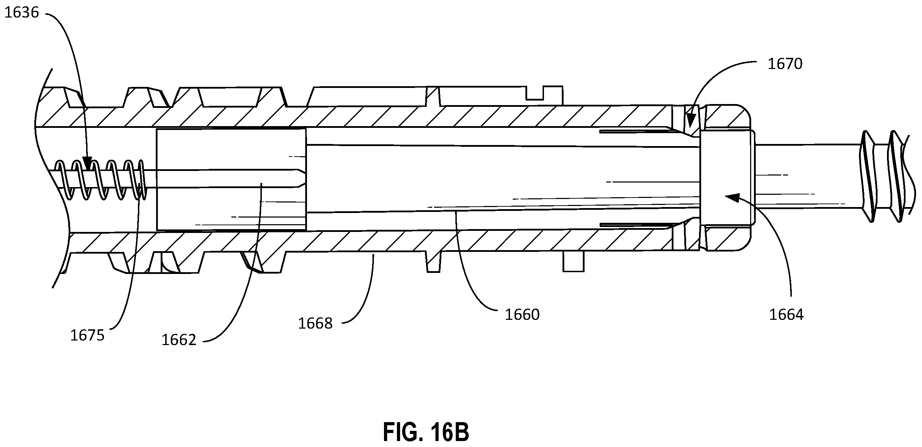

As shown in FIG. 16B, interior shaft 1660 may include rotational fixation protrusion 1662, which may connect to or otherwise interface the threaded shaft such that rotational and/or axial movement of the threaded shaft is translated to interior shaft 1660. Rotational fixation protrusion 1662 may be a rectangular protrusion or any catch or interfacing structure. Interior shaft 1660 may further include proximal protrusion 1664, which may be cylindrical in shape and may have a larger diameter than the body of interior shaft 1660. Proximal protrusion 1664 may interface with snap fit 1670 of a proximal region of threaded shaft to secure interior shaft 1660 to threaded shaft 1668 to prevent interior shaft 1660 from advancing axially beyond snap fit 1670. It is understood that snap fit 1670 may permit proximal protrusion 1664 to traverse snap fit 1670 in a proximal direction but may prevent axial movement with respect to snap fit 1670 in the distal direction once proximal protrusion 1664 traverses snap fit 1670 in the proximal direction.

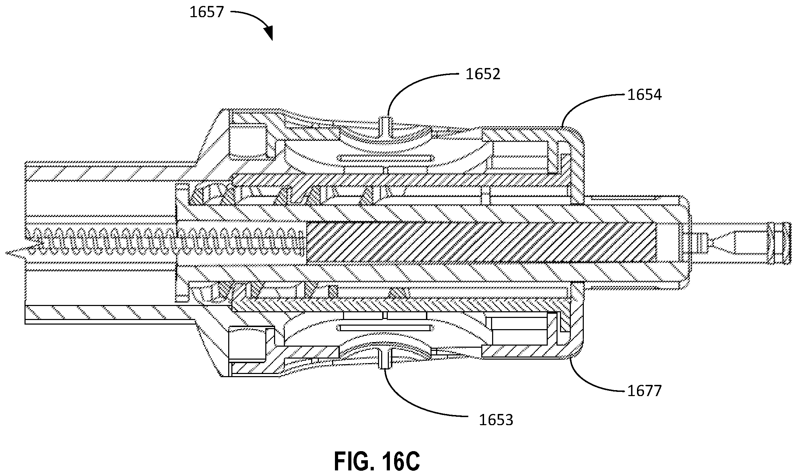

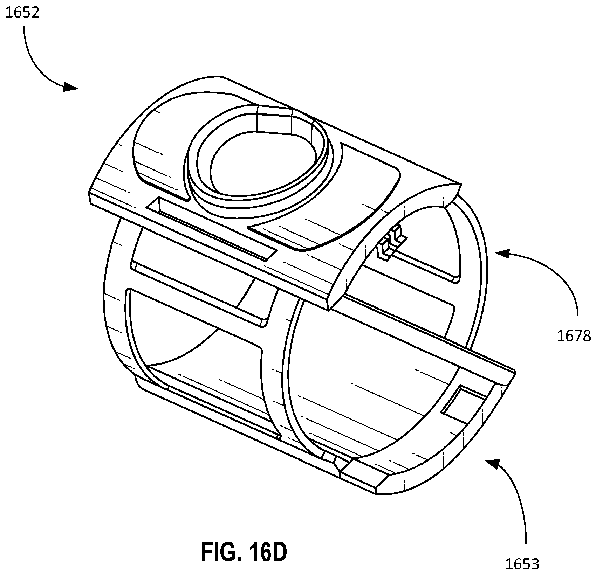

Referring now to FIG. 16C, a cross-sectional view of positioning actuator 1657 is illustrated and may be similar to positioning actuator 1655 of FIG. 16A. Positioning actuator 1657 may include positioning actuator housing 1654, which may include releasing actuator 1652. Positioning actuator 1657 may further include positioning actuator housing 1677, which may be similar to positioning actuator housing 1656, but may further include releasing actuator 1653, which may be the same or similar to releasing actuator 1652. It is understood that releasing actuator 1652 or releasing actuator 1653 may optionally be connected to one another such that movement of releasing actuator 1652 or releasing actuator 1653 causes movement of the other. For example, as shown in FIG. 16D, releasing actuator 1652 and releasing actuator 1653 may be connected via releasing structure 1678, which may be a tubular structure. It is further understood that the positioning shaft may be designed to receive one or both of releasing actuator 1652 and releasing actuator 1653.

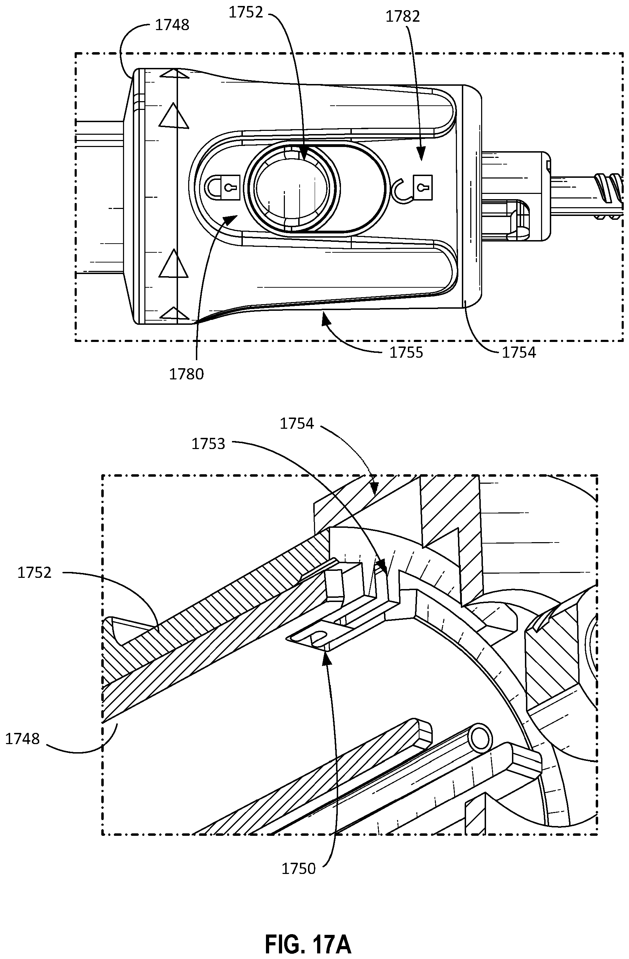

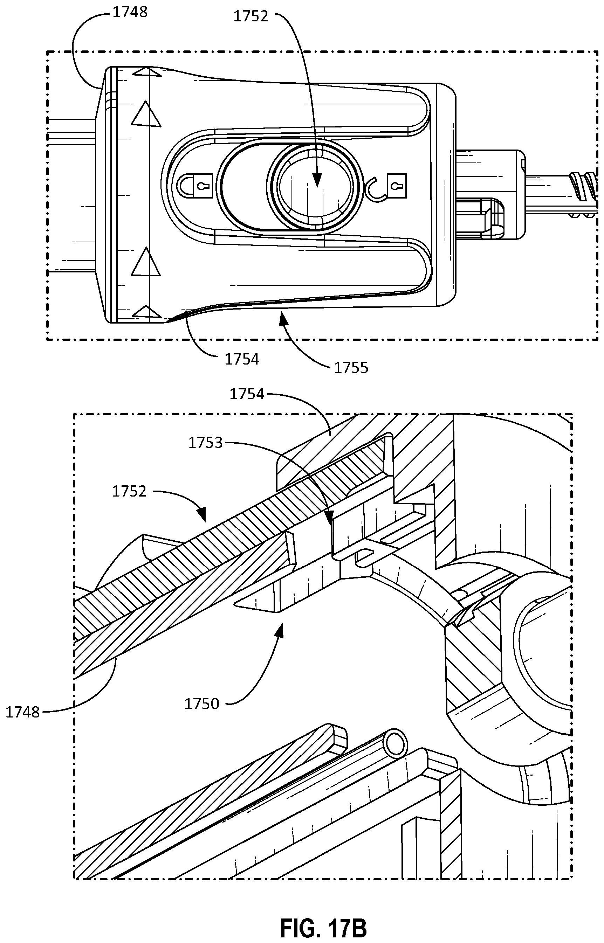

Referring now to FIGS. 17A-17B, locking and unlocking positioning actuator 1755 using releasing actuator 1752 is illustrated. Positioning actuator 1755 may be the same or similar to positioning actuator 1555 of FIG. 15. Positioning actuator 1755 may include positioning actuator housing 1754 that may rotate upon positioning support 1748. Positioning actuator housing 1754 may further include releasing actuator 1752 which may be a button or protrusion that may be engaged by a user and slid along positioning actuator housing 1754 to transition from a locked position, illustrated in FIG. 17A, to an unlocked position, illustrated in FIG. 17B. Positioning actuator housing 1754 may include locked representation 1780 and unlocked representation 1782 to indicate the position of releasing actuator 1752.

As shown in FIG. 17A, releasing actuator 1752 may assume the locked position, which may be distal from the unlocked position. Releasing actuator 1752 may include lock protrusion 1753, which may extend from an inward side of releasing actuator 1752. In the locked position, lock protrusion 1753 of releasing actuator 1752 may be received by receiving area 1750 of positioning support 1748. As shown in FIG. 17A, lock protrusion 1753 may be a rectangular protrusion and receiving area 1750 may also be rectangular in shape. However it is understood that other shapes or designs may be used. In the locked position shown in FIG. 17A, positioning actuator housing 1754 and positioning support 1748 may be caused to rotate together, such that rotation of positioning actuator housing 1754 causes rotation of positioning support 1748.

As shown in FIG. 17B, releasing actuator 1752 may be transitioned to the locked position, proximal from the unlocked position. In the unlocked position, as releasing actuator is moved proximally, locking protrusion 1753 is caused to move proximally, thereby moving locking protrusion 1753 out receiving area 1750 of positioning support 1748, disengaging positioning actuator housing 1754 from positioning support 1748. In the unlocked position, positioning actuator housing 1754 is free to rotate while positioning support 1748 remains stationary.

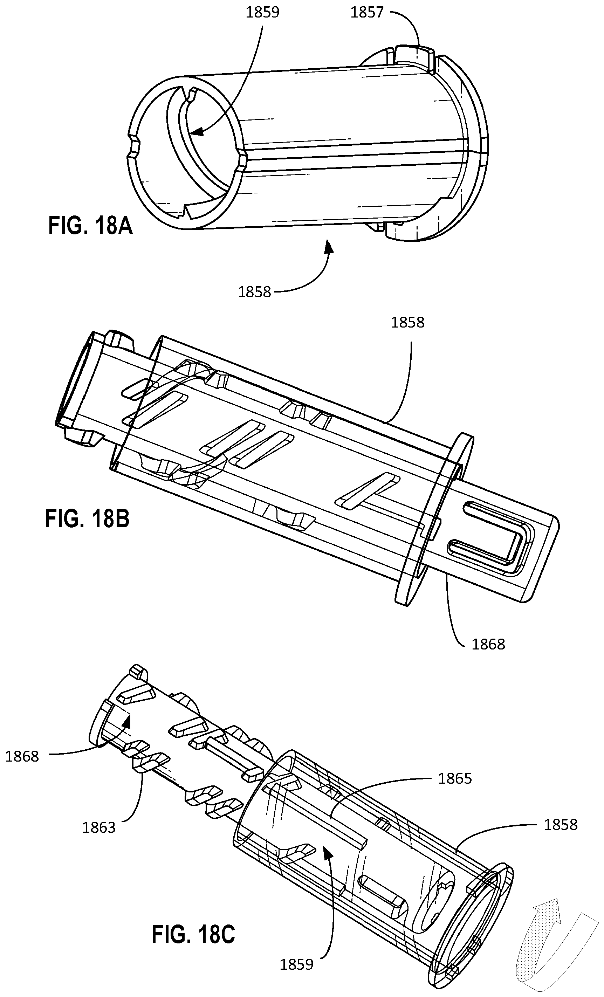

Referring now to FIGS. 18A-18C, actuator base 1858 and threaded shaft 1868 are illustrated. As shown in FIG. 18A, actuator base 1858 may be the same as or similar to actuator base 1558 of FIG. 15 and may include thread 1859 near a distal region of actuator base 1558. Actuator base 1858 may further include proximal protrusion 1857 which may interface with the positioning actuator housing and secure actuator base 1858 to the positioning actuator housing. As shown in FIG. 18B, threaded shaft 1868 may be positioned in actuator base 1858. Threaded shaft 1868 may be the same as or similar to threaded shaft 1568 of FIG. 15. As shown in FIG. 18B, actuator base 1858 may be positioned over a proximal or central region of threaded shaft 1868 in an undeployed position. In the undeployed position, the distal sleeve of the deployment assembly may retain the prosthetic heart valve. As shown in FIG. 18C, threaded shaft 1868 may be advanced proximally by rotating actuator base 1858. As actuator base 1858 is rotated, thread 1859 of actuator base 1858 may engage thread 1863 of threaded shaft 1868 and may cause threaded shaft 1868 to advance distally until thread 1859 of actuator base 1858 engages stop protrusion 1865 which may be a protrusion of threaded shaft 1858 design to prevent any further threaded shaft 1868 with respect to actuator base 1858.

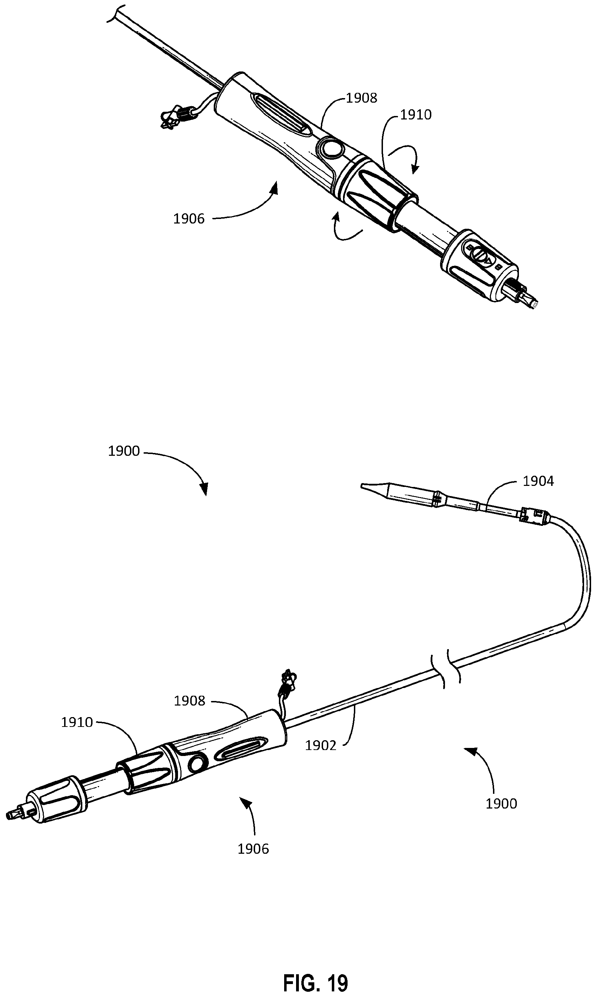

Referring now to FIG. 19, rotating deflection actuator 1910 of catheter system 1900 to cause elongated catheter 1902 to deflect is illustrated. Specially, deflection actuator 1910 of handle 1906 may be rotated by the user. The rotation of deflection actuator 1910 may be with respect to handle body 1908, such that deflection actuator 1910 may be rotated while handle body 1908 remains stationary. As rotation of deflection actuator 1910 causes the deflector wire within handle body 1908 and elongated shaft 1902 to retract proximally, elongated shaft 1902 which is attached to the deflector wire at a distal region is caused to deflect. Such deflection dictates the position and orientation of deployment assembly 1904 at the distal end of elongated shaft 1902. Deflector actuator 1910 may be used to steer elongated shaft 1902 and deployment assembly 1904 through the vasculature of a patient to ultimately deliver a prosthetic heart valve retained by deployment assembly 1904 to a deployment site. For example, deflector actuator 1910 may be used to steer elongated shaft 1902 and deployment assembly 1904 through the aortic arch and to the native aortic valve. It is understood that the degree of curvature in elongated shaft 1902 may be dictated by the degree of rotation and/or number of rotations of deflector actuator 1910.

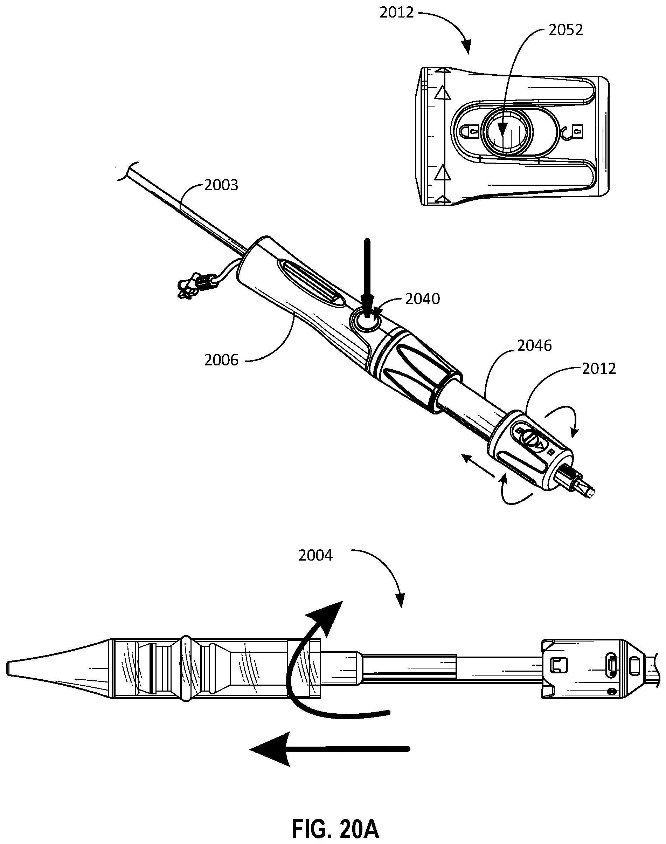

Referring now to FIGS. 20A-20B, manipulation of the positioning actuator of handle 2006 to rotate, advance, and cause deployment of the deployment assembly is illustrated. As shown in FIG. 20A, when positioning actuator 2012, which may be the same as positioning actuator 112 of FIG. 1, is in the locked position such that releasing actuator 2052 is in its most distal position, and unlocking actuator 2040, which may be the same as unlocking actuator 1240 of FIG. 12, is depressed into an unlocked position, rotation of positioning actuator 2012 and/or axial movement of positioning actuator 2012 may be translated to positioning shaft 2046. As positioning shaft 2046 is secured to the torque shaft, rotation of positioning shaft causes rotation of the torque shaft and thus elongated shaft 2003. As deployment assembly 2004 is secured to elongated shaft 2003, rotation and/or axial movement of elongated shaft 2003 is translated to rotational and/or axial movement of deployment assembly 2004. Accordingly, rotational and axial movement of positioning actuator 2012, when positioning actuator 2012 is in the locked position and unlocking actuator 2040 is depressed, results in rotational and/or axial movement of deployment assembly 2004.

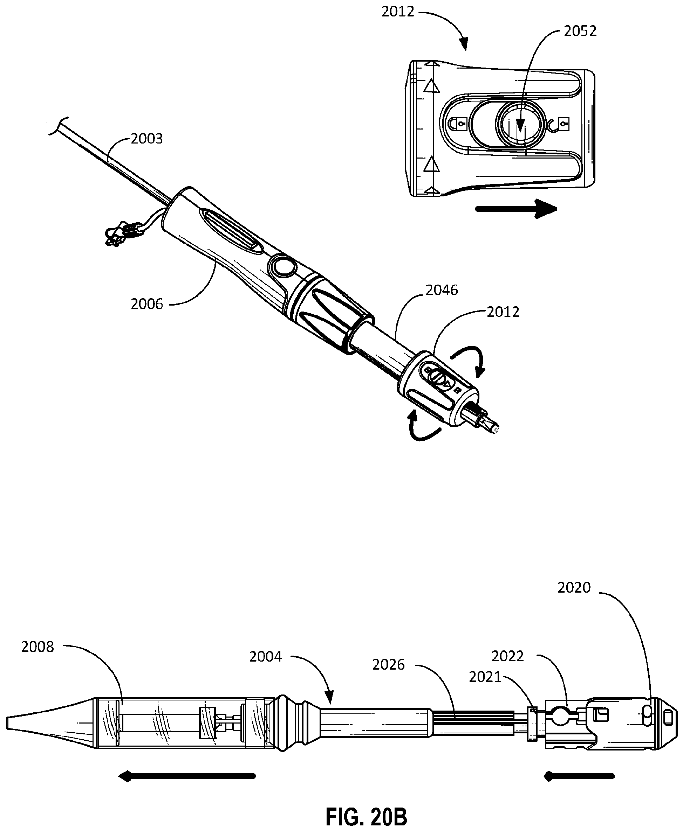

As shown in FIG. 20B, when positioning actuator 2012 is in the locked position such that releasing actuator 2052 is in its most proximal position, rotation of positioning actuator 2012 is translated to the threaded shaft and interior shaft secured to the threaded shaft to cause advancement of the threaded shaft and interior shaft. As the interior shaft is secured to the guidewire shaft, the guidewire shaft is advanced distally with the interior shaft. As the guidewire shaft is secured at the distal end to distal sleeve 2008 of deployment assembly 2004 (e.g., via the end cone), which may be the same as or similar to deployment assembly 304 of FIGS. 3E-3F, distal advancement of the guidewire shaft will cause distal advancement of distal sleeve 2008. Further advancement of distal sleeve will cause distal advancement of anchor connector 2026s which may be secured to anchor support 2022. As anchor connector 2026 advances distally, anchor support 2022 may advance distally from its initial position within proximal sleeve 2020. Accordingly, when releasing actuator 2052 is in the unlocked position, rotation of positioning actuator 2012 may cause distal movement of distal sleeve 2008, internal support 2021 (e.g., tube), and anchor support 2022, thereby deploying the prosthetic valve retained by deployment assembly 2004.

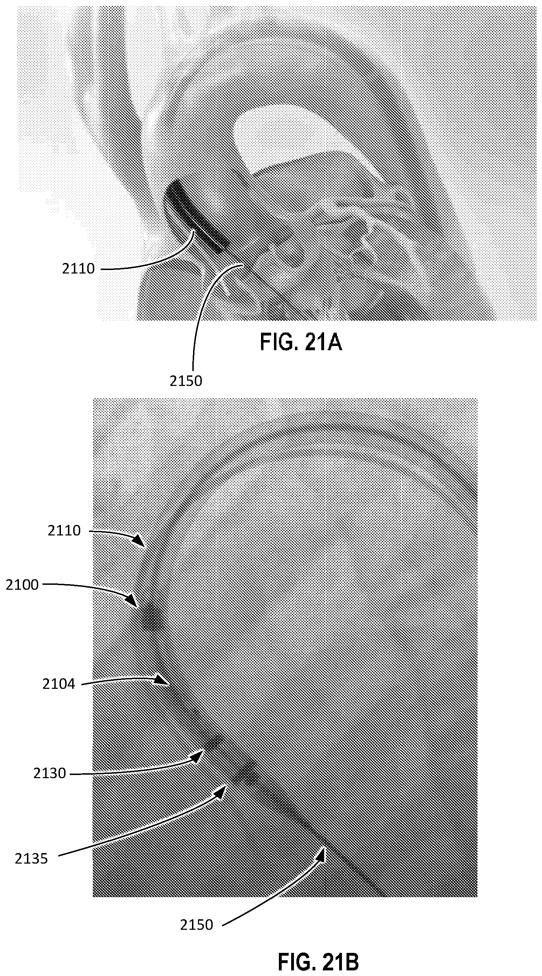

Referring now to FIGS. 21A-21B, initial steps for deploying a catheter system are illustrated. As shown in FIG. 21A, to deploy the catheter system, introducer sheath 2100 may first be introduced to or near the delivery site. For example, guidewire 2150 may be introduced to the patients vasculature and navigated through the vasculature to the deployment site. In one example, a guidewire may be introduced through the femoral artery and navigated through the patient's vasculature to the aortic valve. Next, introducer sheath 2110 may be advanced along guidewire 2150 to or near the implantation site. For example, introducer sheath 2110 may be combined with a dilator (not shown) that may be removed from the introducer sheath upon the introducer sheath reaching the deployment site. As shown in FIG. 21A, it may be desirable for the introducer sheath to be positioned near the delivery site (e.g., on the outflow side of the aortic valve).

Once the introducer sheath is positioned at the delivery site, catheter system 2100, which may be the same as or similar to catheter system 100 of FIG. 1, may be delivered to the delivery site using introducer sheath 2100 as shown in FIG. 21B. Specifically, catheter system 2100 may be positioned within introducer sheath 2110 and travel through introducer sheath 2110 to the delivery site. Deployment assembly 2104 may further include imaging marker 2130 which may appear using well known medical imaging. For example, imaging marker 2130 may be a ring positioned on a proximal region of the distal sleeve deployment assembly 2104. Similarly the distal end of introducer sheath 2110 may include imaging marker 2135 identifying the end of introducer sheath 2135. It may be helpful for an individual delivering catheter system 2100 to the implantation site to know when the prosthetic heart valve is exiting an introducer sheath 2110 and/or nearing the end of introducer sheath 2110. In one example, imaging marker 2130 and/or imaging marker 2135 may be a radiopaque or other well-known imaging marker.

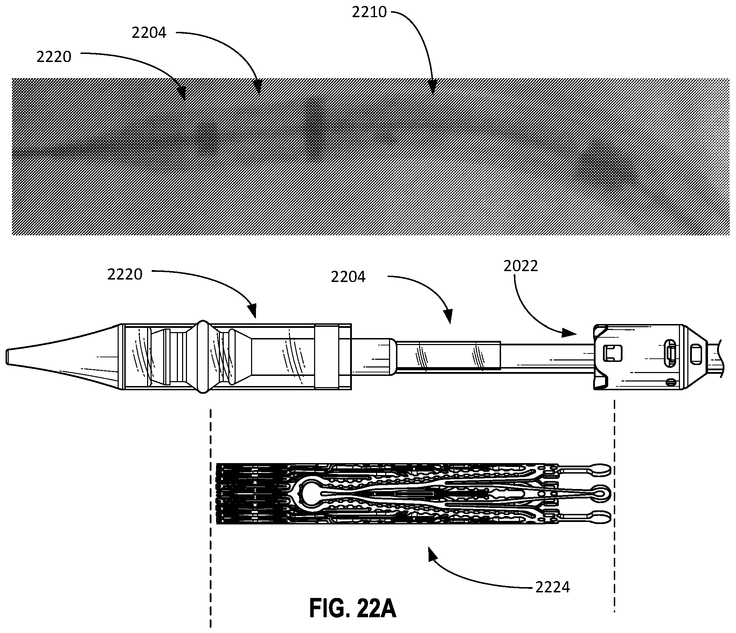

Referring now to FIGS. 22A-22D, sequential deployment of the prosthetic heart valve is illustrated. Referring now to FIG. 22A, deployment assembly 2204, which may be the same as deployment assembly 2104 of FIG. 21B, is illustrated at the distal end introducer sheath 2210, which may be the same as introducer sheath 2110 of FIG. 21B. Deployment assembly 2204 may be positioned such that a proximal portion of distal sleeve 2220 is still retained by introducer sheath 2210. In this position, prosthetic heart valve 2224 may be fully constrained and compressed by introducer sheath 1210 and deployment assembly 2204. As shown in FIG. 22A, distal sleeve 2220 and anchor support 2022 may be in their proximal most position.

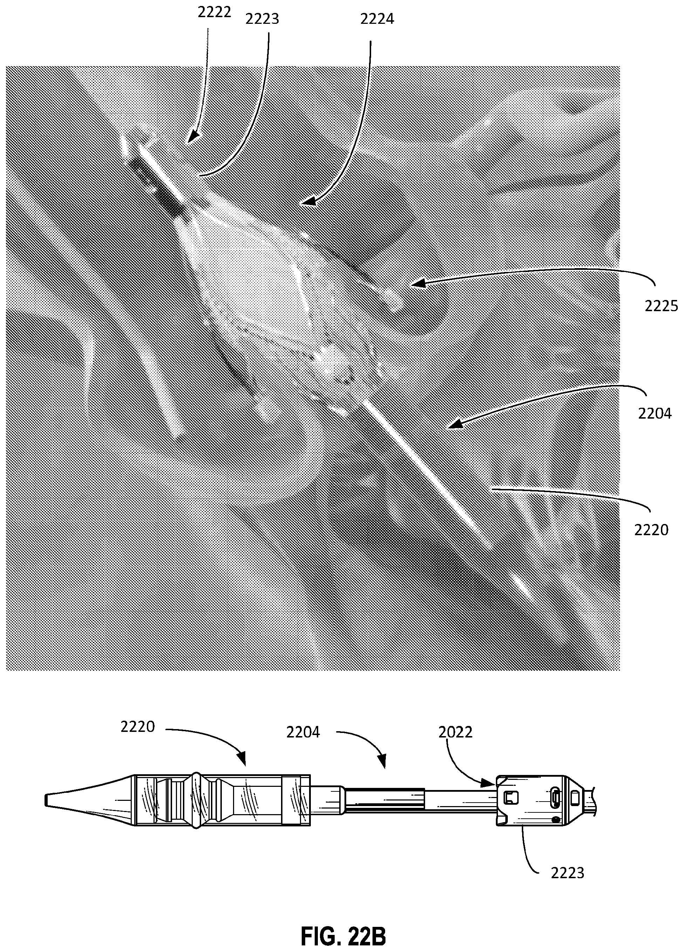

Referring now to FIG. 22B, deployment assembly 2204 is shown after fully exiting the introducer sheath. Upon existing the introducer sheath, positioning arms 2225 of prosthetic heart valve 2224 may be permitted to expand and deployment assembly 2204 may be rotated and/or advanced such that positioning arms 2225 may be positioned into native cusps of the native valve (e.g., aortic valve). As shown in FIG. 22B, upon deployment assembly 2204 exiting the introducer sheath, distal sleeve 2220 of deployment assembly 2204 may be in its proximal most positon and thus may retain a distal portion of prosthetic heart valve 2224 in a compressed state. Also, upon deployment assembly 2204 exiting the introducer sheath, anchor support 2222 may be in its proximal most position within proximal sleeve 2223 and thus may maintain the proximal portion of prosthetic heart valve 2224 in a compressed state. Accordingly, while the proximal portion and distal portions of prosthetic heart valve 2224 are maintained in a compressed state, positioning arms 2225 are permitted to at least partially expand.

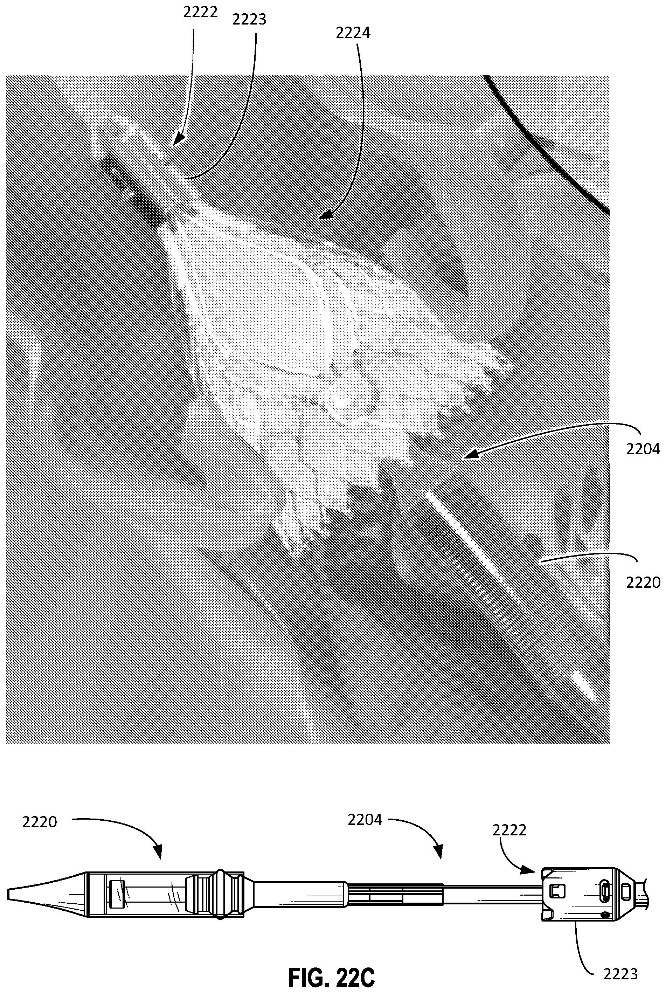

Referring now to FIG. 22C, deployment assembly 2204 is shown with its distal portion in an expanded state but its proximal portion retained in a compressed state. As shown in FIG. 22C, the retaining arms of prosthetic heart valve 2224 may be positioned in the cusps of the native leaflets of the aortic valve, similar to FIG. 22B. However, distal sleeve 2220 of deployment assembly 2204 may move distally, thereby releasing the distal portion of prosthetic heart valve 2224 such that the distal portion of prosthetic heart valve 2224 may transition to an expanded state. In in the expanded state, the distal portion of prosthetic heart valve 2224 may clip or otherwise sandwich a portion of the native leaflet between the positioning arms and the distal portion of prosthetic heart valve 2224, therby anchoring prosthetic heart valve 2224 to the native valve. Though distal sleeve 2220 is moved distally, anchor support 2222 may remain in its proximal most position within proximal sleeve 2223. Accordingly, the proximal portion of prosthetic heart valve may remain in a compressed state while the distal portion of the prosthetic heart valve is permitted to transition to an expanded state.

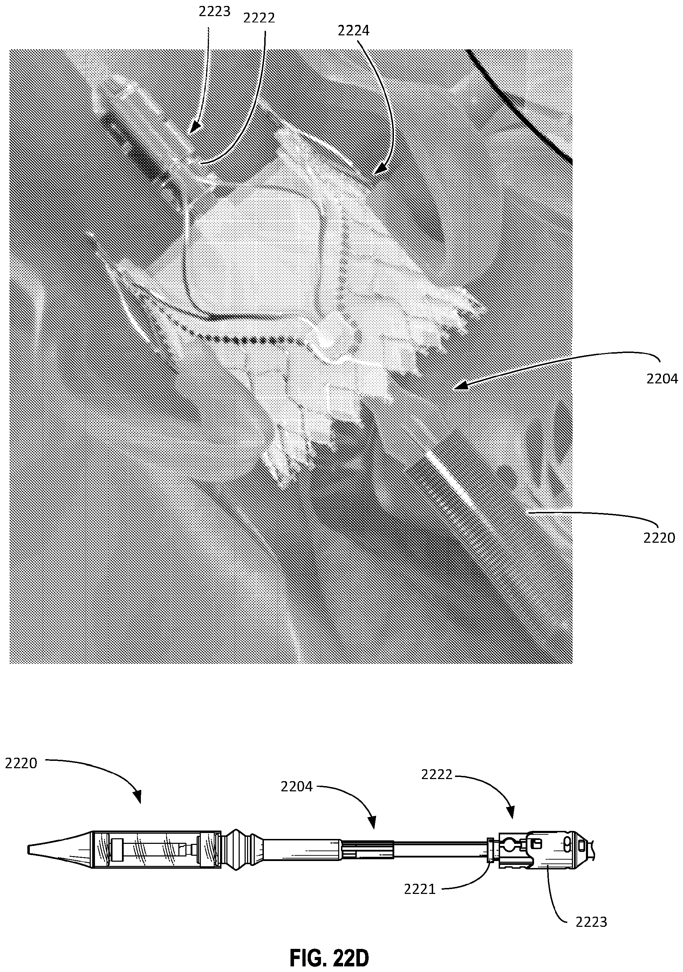

Referring now to FIG. 22D, the deployment assembly is shown with both the distal sleeve and anchor support in their distal most positions. As shown in FIG. 22D, distal sleeve 2220 may continue to move distally to a distal most position and as distal sleeve 2220 moves distally, distal sleeve 2220 and/or the end cone may engage anchor connector and cause anchor support 2222 and/or internal support 2221 (e.g., tube) to similarly move distally, causing anchor support 2222 to exit proximal sleeve 2223. As anchor support 2222 moves distally from proximal sleeve 2223, a proximal portion of prosthetic heart valve 2224 may be released from proximal sleeve 2223 and anchor support 2222 and may be permitted to transition to an expanded state, such that prosthetic heart valve 2224 may be transitioned to a fully expanded state. It is understood that as the proximal portion of prosthetic heart valve 2224 transitions to an expanded state, the compression force on the native leaflet positioned between the positioning arms and the distal region of the prosthetic heart valve may be increased, further securing the prosthetic heart valve to the native valve (e.g., aortic valve).

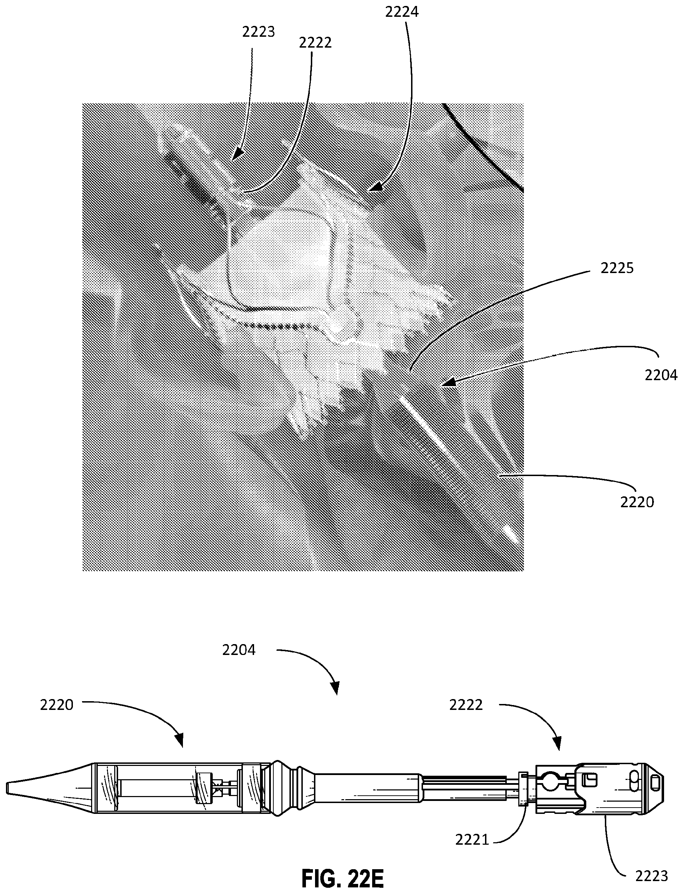

Referring now to FIG. 22E, deployment assembly is 2204 is shown is its retraction position. As shown in FIG. 22E, distal sleeve 2200 may be moved proximally after achieving its distal most position to cause the proximal open end of distal sleeve 2200 to engage retraction taper assembly 2225 of deployment assembly 2204. It is understood that by causing the proximal open end of distal sleeve 2200 to engage retraction taper assembly 2225, deployment assembly 2204 may be removed from prosthetic heart valve 2224 and the vasculature of the patient without the proximal open end of distal sleeve 2200 damaging the vasculature or other tissue of the patient or prosthetic heart valve 2224. Optionally, anchor support 2222 and/or internal support (e.g., tube) may further be moved distally back into proximal sleeve 2223 to prevent anchor support 2222 from damaging the vasculature or other tissue of the patient or prosthetic heart valve 2224.

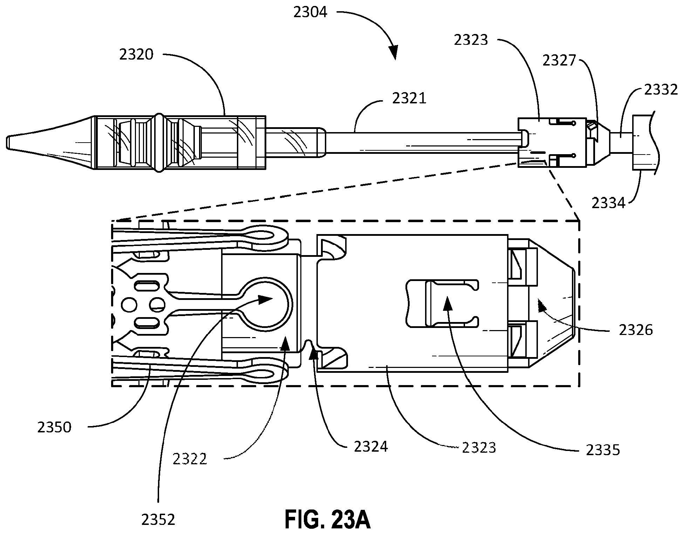

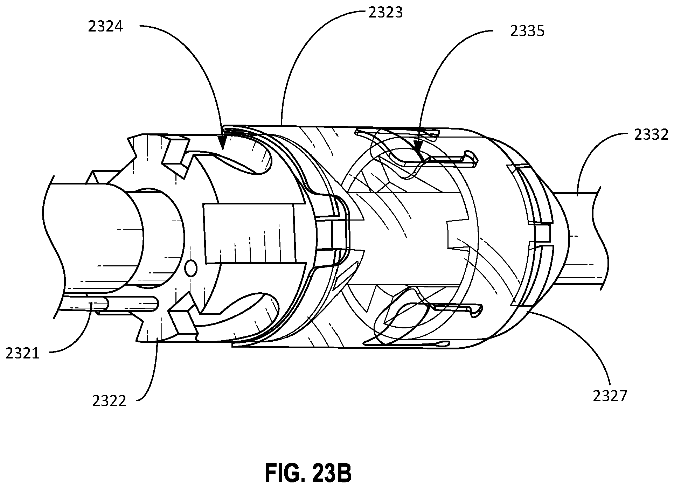

Referring now to FIGS. 23A-23B, an exemplary anchor support and proximal sleeve are illustrated. As shown in FIGS. 23A-23B, deployment assembly 2304 may include distal sleeve 2320, proximal sleeve 2323, anchor support 2322, and proximal taper 2327. Proximal taper 2327 may be secured to elongated shaft 2332, including the torque tube. Proximal taper 2327 may be secured to proximal sleeve 2323, which may include one or more tabs 2335. Proximal sleeve 2323 may be tubular in shape and tab 2335 may be cantilevered such that a distal end of each tab 2335 may deflect. Anchor support 2322 may be sized to fit within proximal sleeve 2323 and may be selectively advanced distally such that at least a portion of anchor support 2322 exits proximal sleeve 2323, as shown in FIGS. 23A-23B. Anchor support 2322 may include recessed portions 2334, which may be recessed or otherwise cutaway from an outer surface of anchor support 2322. Recessed portions 2324 may be shaped to receive anchors 2352 of prosthetic heart valve 2350. For example, anchors 2352 may be circular in shape and recessed portions 2324 may similarly be in circular in shape with a slightly larger diameter and a recessed depth such that anchors 2352 may fit between anchor support 2322 and proximal sleeve 2323. It is understood, however, that recessed portions 2324 may be any other shape (e.g., rectangular).

Tabs 2335 may be designed to interface with anchors 2352 such that tabs 2335 may resist distal movement of anchors 2335 and/or anchor support 2324. Introducer sheath 2334 may be designed to interface with tabs 2335 such that introducer sheath may apply a downward force on tabs 2335 as deployment assembly 2304 traverses introducer sheath 2334. When proximal sleeve 2323 is within introducer sheath 2334, tabs 2335 may prevent anchor support 2324 from prematurely moving distally. Once proximal sleeve 2323 and tabs 2335 exit introducer sheath 2334, tabs 2335 may permit anchor support 2322 to exit proximal sleeve 2323 once distal sleeve applies a distal force on anchor support 2322 via anchor connector 2321.

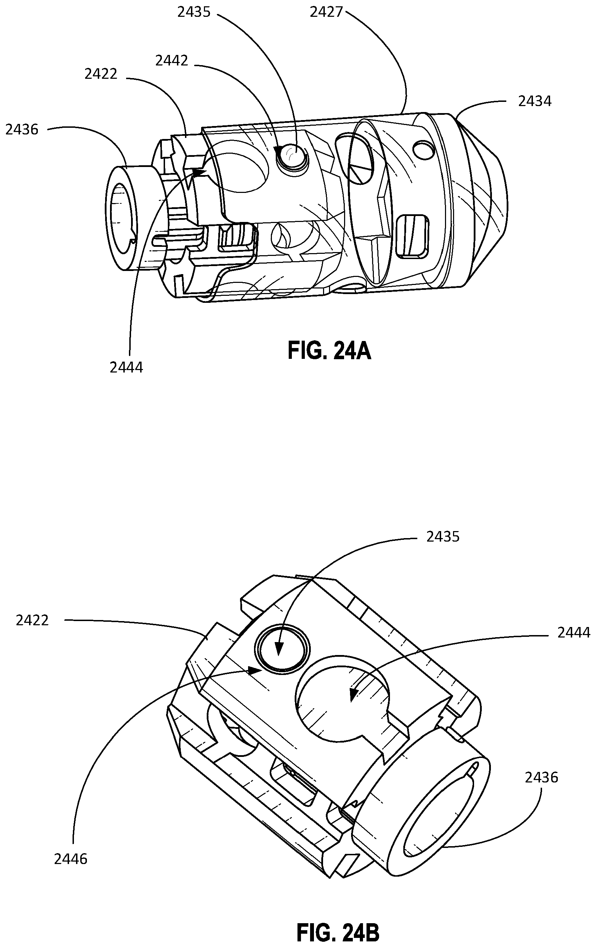

Referring now to FIGS. 24A-24E, an alternative anchor support and proximal sleeve are illustrated. This anchor support and sleeve may be incorporated into the deployment assemblies of the embodiments described above to permit locking of features together. As shown in FIGS. 24A-24B, anchor support 2422 may be tubular in shape. The deployment assembly may include lock 2435 for locking proximal sleeve 2427 to anchor support 2422 during delivery. The handle at the proximal end, when actuated, causes lock 2435 of the deployment assembly to unlock such that anchor support 2422 is longitudinally moveable relative to sleeve 2427 for expanding and implanting the prosthetic heart valve.

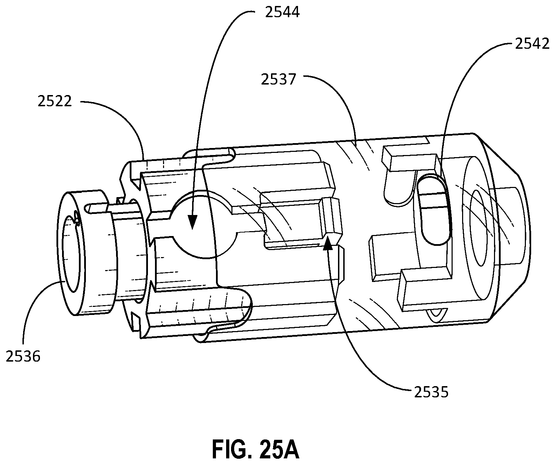

Lock 2435 may include a protrusion and a receptacle. The protrusion extends into the receptacle to lock sleeve 2427 to anchor support 2422 and is released from the receptacle to unlock sleeve 2427 from anchor support 2422. As illustrated, anchor support 2422 may have the protrusion and proximal sleeve 2427 may have the receptacle. In the embodiment shown in FIG. 24A, the protrusion is a ball bearing. Alternatively, as shown in FIGS. 25A-25D discussed below, the protrusion may be a lever that is held in the receptacle in a snapping manner when locked and released from the receptacle when unlocked. In some embodiments, there are multiple protrusions and receptacles for the locking the anchor support with respect to the proximal sleeve. As illustrated, there may be multiple locking balls or levers spaced around the circumference.

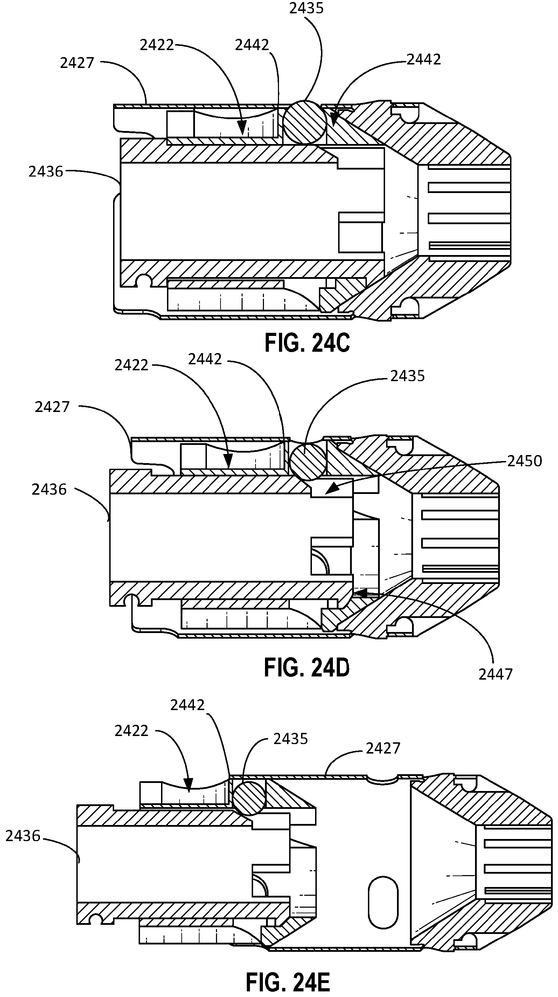

Lock 2435 may further interface with tube/internal support 2436 that, in a first position (shown in FIG. 24C), causes the protrusion to extend into the receptacle. Tube/internal support 2436 moves relative to anchor sleeve 2422 to a second position (shown in FIG. 24E) to release the protrusion from the receptacle.

Anchor support 2422 may fit within proximal sleeve 2327, which also may be tubular in shape. Anchor support 2422 may include recessed portions 2444 which may be recessed or otherwise cutaway from an outer surface of anchor support 2422. Recessed portions 2444 may be shaped to receive anchors of a prosthetic heart valve. For example, anchors may be circular in shape and recessed portions may similar be in circular in shape. It is understood that recessed portions 2444 may be any other shape (e.g., rectangular).

Anchor support 2422 may further include through-hole 2446, which may be circular in shape and may permit lock 2435 to traverse through anchor support 2422 in through-hole 2446. Lock 2435 may be a ball structure or ball stopper (e.g., a plastic, rubber, or metallic ball, or the like). Proximal sleeve 2427 may similarly include through hole 2442 (e.g., a receptacle) which may be circular in shape but may have a diameter smaller than lock 2435 such that only a portion of lock 2435 may extend through through-hole 2442. Distal taper 2434 may be secured to a proximal end of proximal sleeve 2427. Internal support 2436 may be a tubular structure sized to fit within anchor support 2422. Internal support may support lock 2435 and maintain lock 2435 in a locked position when internal support 2436 is in its most proximal position. Internal support 2436 may advance distally with respect to anchor support 2422 and proximal sleeve 2427 and as a result, lock 2435 may no longer maintain lock 2435 in a locked position. When internal support 2436 advances distally, lock may be permitted to drop (e.g., move inwardly), permitting anchor support 2422 to move distally with respect to proximal sleeve 2427 as lock 2435 no longer interfaces with proximal sleeve 2427. As anchor support 2422 moves distally with respect to proximal sleeve 2427, recessed portions 2444 may exit proximal sleeve 2427 and may release the anchors of the prosthetic heart valve. In this manner, lock 2435 may either prevent or permit axial movement of anchor support 2422 and thus deployment of the anchors. While only one lock 2435 is illustrated, it is understood that multiple locks 2435 may be used and may be circumferentially spaced apart on anchor support 2436.

Internal support 2436 moving distally and permitting anchor support 2422 to move distally is illustrated in FIGS. 24C-24E. As shown in FIG. 24C, internal support 2436 may be in its proximal most position with respect to anchor support 2422 and proximal sleeve 2427. As shown in FIG. 24C, in the proximal-most position of internal support 2436, lock 2435 may be supported by an outer surface of internal support 2436, such that lock 2435 rests within through-hole 2442 and extends partially through through-hole 2442 of proximal sleeve 2427. In this manner, lock 2435 may prevent anchor support 2436 from moving distally while internal support 2436 is in its proximal-most positon and lock 2435 is interfacing with proximal sleeve 2427.

Shown in FIG. 24D, internal support 2436 may be moved distally while anchor support 2422 and proximal sleeve 2327 remains stationary. For example, the anchor connector connected to the distal sleeve and/or the end cone may be connected to internal support 2436 and not directly to anchor support 2422. Internal support 2436 may include catch 2447, which may be a protrusion that extends outward from the outer surface of internal support 2436 and may interface with anchor support 2422 after internal support 2436 has moved a certain amount distally. Once catch 2447 contacts anchor support 2422, anchor support 2422 and internal support 2436 will move distally together. Internal support 2436 may further include lock recess 2450, which may be a recess or cutout of the outer surface of a proximal region of internal support 2436, which may be sized to permit lock 2435 to at least partially enter. Lock recess 2450 may be sloped at is proximal end, to permit lock 2435 to gradually enter. As shown in FIG. in FIG. 24D, as internal support 2436 is moved distally with respect to anchor support 2422 and proximal sleeve 2427, lock recess 2450 is positioned beneath lock 2435 and lock 2435 is permitted to enter lock recess 2450. As lock 2435 enters lock recess 2450, lock 2435 falls below proximal sleeve 2427, permitting anchor support 2436 to move distally with respect to proximal sleeve 2427, as shown in FIG. 24E. In this manner, the recessed portions of anchor support 2522 may be exposed from proximal sleeve 2437 to permit at least the proximal portion of the prosthetic heart valve to transition to an expanded state.

Referring now to FIGS. 25A-25D, an alternative anchor support and proximal sleeve are illustrated. As shown in FIG. 25A-25D, anchor support 2522 may be tubular in shape. Anchor support 2522 may fit within proximal sleeve 2537, which also may be tubular in shape. Anchor support 2522 may include recessed portions 2544, which may be recessed or otherwise cutaway from an outer surface of anchor support 2522. Recessed portions 2544 may be shaped to receive anchors of a prosthetic heart valve. Anchor support 2522 may further include cantilevered lock 2525 (e.g., a lock), which may be cantilevered and free standing at a proximal end of anchor support 2422. For example, cantilevered lock 2525 may be a lever. Cantilevered lock 2435 may lock anchor support 2522 to proximal sleeve 2537 during delivery. Cantilevered lock 2535 have elastic properties and may deflect downward. Cantilevered lock 2535 may extend outward beyond the surface of anchor support 2522 and may be designed to resist movement in the distal direction. Proximal sleeve may further include through-hole 2542 which may be a receptacle sized and shaped to receive at least a portion of cantilever lock 2535. Internal support 2536 (e.g., tube) may advance distally with respect to anchor support 2522 and proximal sleeve 2527 and as a result, cantilevered lock 2535 may transition from a locked position to an unlocked position. In this manner, cantilevered lock 2535 may either prevent or permit axial movement of anchor support 2522. While only one cantilever lock 2535 is illustrated, it is understood that multiple cantilevered locks 2535 may be used and may be circumferentially spaced apart on anchor support 2522.

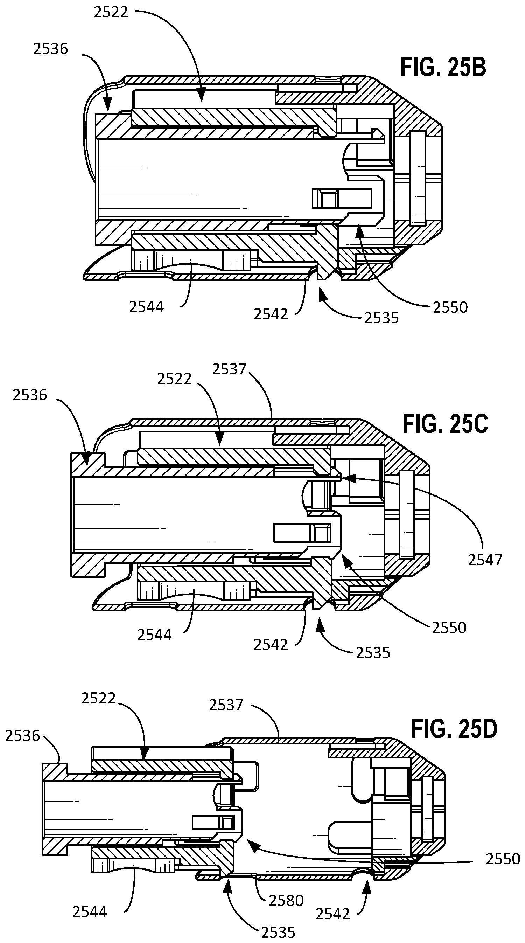

Internal support 2536 moving distally and permitting anchor support 2522 to move distally is illustrated in FIGS. 25B-25D. As shown in FIG. 25B, internal support 2536 may be in its distal-most position with respect to anchor support 2522 and proximal sleeve 2527. As shown in FIG. 25B, in the proximal-most position of anchor support 2536, cantilevered lock 2535 may be supported at a proximal end by an outer surface of internal support 2536, such that cantilevered lock 2535 extends at least partially through through-hole 2542 of proximal sleeve 2537. In this manner, cantilevered lock 2535 may prevent anchor support 2522 from moving distally while internal support 2536 is in its proximal-most positon.

As shown in FIG. 25C, internal support 2536 may be advanced distally while anchor support 2522 and proximal sleeve 2527 remain stationary. For example, the anchor connector connected to the distal sleeve and/or end cone may be connected to internal support 2536 and not directly to anchor support 2522. Internal support 2536 may include catch 2547, which may be a protrusion that extends outward from the outer surface of internal support 2536 and may interface with anchor support 2522 after internal support 2436 has moved a certain amount distally. Once catch 2547 contacts anchor support 2522, anchor support 2522 and internal support 2536 will move distally together.

Internal support 2536 may further include lock recess 2550, which may be a recess or cutout of the outer surface of a proximal region of internal support 2536, which may be sized to permit lock 2435 to at least partially deflect or otherwise extend into lock recess 2450. It is understood that cantilevered lock 2535 may be biased downward, such that a proximal end of cantilevered lock 2535 is biased downward toward internal support 2536. Lock recess 2450 may be sloped at is distal end to permit cantilevered lock 2535 to gradually deflect or otherwise extend into lock recess 2550. As shown in FIG. 25C, as internal support 2536 is moved distally with respect to anchor support 2522 and proximal sleeve 2527, lock recess 2550 is positioned beneath cantilevered lock 2535 and cantilevered lock 2535 is permitted to deflect or otherwise extend into lock recess 2550. As cantilevered lock 2535 deflects into or otherwise extends into lock recess 2550, cantilevered lock 2535 may be positioned below proximal sleeve 2527, permitting anchor support 2536 to move distally with respect to proximal sleeve 2527, as shown in FIG. 25D. In this manner, the recessed portions of anchor support 2522 may be exposed from proximal sleeve 2537 to permit at least the proximal portion of the prosthetic heart valve to transition to an expanded state. Proximal sleeve 2535 may further include distal through-hole 2580 in which cantilevered lock 2535 may enter in its distal most position, such that distal through-hole 2580 may prevent further distal movement anchor support 2522.
The foregoing description of illustrative embodiments has been presented for purposes of illustration and of description. It will of course be understood that the embodiments described herein are illustrative, and components may be arranged, substituted, combined, and designed in a wide variety of different configurations.

## Claims

1. A catheter system (100, 1900, 2100) for implanting a prosthetic heart valve, the catheter system (100, 1900, 2100) comprising:
- an elongated shaft (102, 202, 302, 402, 1102, 1902, 2003, 2332) comprising a proximal region and a distal region;
- a deployment assembly (104, 304, 1904, 2004, 2204, 2304) at the distal region of the elongated shaft (102, 202, 302, 402, 1102, 1902, 2003, 2332), the deployment assembly (104, 304, 1904, 2004, 2304) sized and shaped to be advanced to an implantation site at a native heart valve site with the prosthetic heart valve in a collapsed state, the deployment assembly (104, 304, 1904, 2004, 2304) comprising a sleeve (320, 2020, 2223, 2323, 2327, 2427, 2527) configured to maintain at least a portion of the prosthetic heart valve in the collapsed state within the sleeve (320, 2020, 2223, 2323, 2327, 2427, 2527) during delivery; and
- a handle (106, 206, 1106, 1906, 2006) disposed at a proximal region of the elongated shaft (102, 202, 302, 402, 1102, 1902, 2003, 2332), the handle (106, 206, 1106, 1906, 2006) comprising a handle body (108, 208, 1108, 1408, 1908), a first actuator (112, 212, 1112, 1555, 1655, 1755) configured to be rotated relative to the handle body (108, 208, 1108, 1408, 1908), and a second actuator (224, 1552, 1652, 1752) configured to transition between a first position and a second position,
wherein, when the second actuator (224, 1552, 1652, 1752) is in the first position, rotation of the first actuator (112, 212, 1112, 1555, 1655, 1755) is translated to the deployment assembly (104, 304, 1904, 2004, 2304) to cause the deployment assembly (104, 304, 1904, 2004, 2304) to rotate and, when the second actuator (224, 1552, 1652, 1752) is in the second position, rotation of the first actuator (112, 212, 1112, 1555, 1655, 1755) causes the sleeve (320, 2020, 2223, 2323, 2327, 2427, 2527) to move longitudinally relative to the handle (106, 206, 1106, 1906, 2006) for expanding and implanting the prosthetic heart valve.

2. The catheter system (100, 1900, 2100) of claim 1,
wherein the handle (106, 206, 1106, 1906, 2006) comprises a third actuator (211, 1140, 1240, 1340, 2040) that, when in a first position, prevents movement of the first actuator (112, 212, 1112, 1555, 1655, 1755) when the second actuator (224, 1552, 1652, 1752) is in the first position and, when in a second position, permits movement of the first actuator (112, 212, 1112, 1555, 1655, 1755) when the second actuator (224, 1552, 1652, 1752) is in the first position.

3. The catheter system (100, 1900, 2100) of claim 2,
wherein the handle (106, 206, 1106, 1906, 2006) comprises a fourth actuator (110, 210, 1110, 1910) configured to rotate relative to the handle body (108, 208, 1108, 1408, 1908) and independent of the first actuator (112, 212, 1112, 1555, 1655, 1755), the fourth actuator (110, 210, 1110, 1910) connected to a distal portion of the elongated shaft (102, 202, 302, 402, 1102, 1902, 2003, 2332) and configured to cause the elongated shaft (102, 202, 302, 402, 1102, 1902, 2003, 2332) to deflect.

4. The catheter system (100, 1900, 2100) of claim 1,
wherein, when the second actuator (224, 1552, 1652, 1752) is in the second position, rotation of the first actuator (112, 212, 1112, 1555, 1655, 1755) does not cause the deployment assembly (104, 304, 1904, 2004, 2304) to rotate and, when the second actuator (224, 1552, 1652, 1752) is in the first position, rotation of the first actuator (112, 212, 1112, 1555, 1655, 1755) does not cause the sleeve (320, 2020, 2223, 2323, 2327, 2427, 2527) to move longitudinally relative to the handle (106, 206, 1106, 1906, 2006).

5. The catheter system (100, 1900, 2100) of claim 1,
wherein the deployment assembly (104, 304, 1904, 2004, 2304) further comprises a second sleeve (312, 902, 2008, 2220) proximal to the sleeve (320, 2020, 2223, 2323, 2327, 2427, 2527) and coupled to the elongated shaft (102, 202, 302, 402, 1102, 1902, 2003, 2332) and an anchor support (322, 2022, 2222, 2322, 2422, 2522) positioned within the second sleeve (312, 902, 2008, 2220) and in mechanical communication with the sleeve (320, 2020, 2223, 2323, 2327, 2427, 2527).

6. The catheter system (100, 1900, 2100) of claim 5,
wherein the anchor support (322, 2022, 2222, 2322, 2422, 2522) is configured to receive a proximal portion of the prosthetic valve and the anchor support (322, 2022, 2222, 2322, 2422, 2522) and the second sleeve (312, 902, 2008, 2220) are configured to retain the proximal portion of prosthetic heart valve in a compressed state.

7. The catheter system (100, 1900, 2100) of claim 5,
wherein longitudinal movement of the sleeve (320, 2020, 2223, 2323, 2327, 2427, 2527) causes longitudinal movement of the anchor support (322, 2022, 2222, 2322, 2422, 2522).

8. The catheter system (100, 1900, 2100) of claim 1,
wherein when the second actuator (224, 1552, 1652, 1752) is in the first position, the first actuator (112, 212, 1112, 1555, 1655, 1755) is rigidly connected to the deployment assembly (104, 304, 1904, 2004, 2304).

9. The catheter system (100, 1900, 2100) of claim 1,
wherein the second actuator (224, 1552, 1652, 1752) comprises a protrusion and when the second actuator (224, 1552, 1652, 1752) is in the first position, the protrusion of the second actuator (224, 1552, 1652, 1752) is configured to engage a shaft (1144, 1247, 1347, 1444, 1568, 1668, 1868) disposed within the handle (106, 206, 1106, 1906, 2006) and rigidly connected to the deployment assembly (104, 304, 1904, 2004, 2304).

10. The catheter system (100, 1900, 2100) of claim 9,
wherein the shaft (1144, 1247, 1347, 1444, 1568, 1668, 1868) is threaded and a cross-section of the shaft (1144, 1247, 1347, 1444, 1568, 1668, 1868) comprises at least one right angle.

11. The catheter system (100, 1900, 2100) of claim 10,
wherein the handle (106, 206, 1106, 1906, 2006) further comprises a third actuator (211, 1140, 1240, 1340, 2040) configured to interface with one or more threads (910, 1566, 1669) of the shaft (1144, 1247, 1347, 1444, 1568, 1668, 1868) and the at least one right angle to selectively restrain the deployment assembly (104, 304, 1904, 2004, 2304) from axial and rotational movement.

12. The catheter system (100, 1900, 2100) of claim 11,
wherein the third actuator (211, 1140, 1240, 1340, 2040) comprises a depressible body having a central channel and a ridged wheel (1244) disposed within the central channel and configured to receive and rotate with the shaft (1144, 1247, 1347, 1444, 1568, 1668, 1868), and wherein the third actuator (211, 1140, 1240, 1340, 2040) is configured to resist rotation of the ridged wheel (1244) in a locked position.

## Patentansprüche

1. Kathetersystem (100, 1900, 2100) zum Implantieren einer Herzklappenprothese, wobei das Kathetersystem (100, 1900, 2100) umfasst:
- einen länglichen Schaft (102, 202, 302, 402, 1102, 1902, 2003, 2332), der einen proximalen Bereich und einen distalen Bereich umfasst;
- eine Entfaltungsanordnung (104, 304, 1904, 2004, 2204, 2304) an dem distalen Bereich des länglichen Schafts (102, 202, 302, 402, 1102, 1902, 2003, 2332), wobei die Entfaltungsanordnung (104, 304, 1904, 2004, 2304) bemessen und geformt ist, um mit der Herzklappenprothese in einem zusammengefalteten Zustand zu einer Implantationsstelle an einer Stelle einer nativen Herzklappe vorgeschoben zu werden, wobei die Entfaltungsanordnung (104, 304, 1904, 2004, 2304) eine Hülse (320, 2020, 2223, 2323, 2327, 2427, 2527) umfasst, die dazu eingerichtet ist, zumindest einen Abschnitt der Herzklappenprothese in dem zusammengefalteten Zustand innerhalb der Hülse (320, 2020, 2223, 2323, 2327, 2427, 2527) während der Zuführung zu halten; und
- einen Handgriff (106, 206, 1106, 1906, 2006), der an einem proximalen Bereich des länglichen Schafts (102, 202, 302, 402, 1102, 1902, 2003, 2332) angeordnet ist, der Handgriff (106, 206, 1106, 1906, 2006) umfassend einen Handgriffkörper (108, 208, 1108, 1408, 1908), ein erstes Betätigungselement (112, 212, 1112, 1555, 1655, 1755), das dazu eingerichtet ist, relativ zu dem Handgriffkörper (108, 208, 1108, 1408, 1908) gedreht zu werden, und ein zweites Betätigungselement (224, 1552, 1652, 1752), das dazu eingerichtet ist, zwischen einer ersten Position und einer zweiten Position zu wechseln, wobei, wenn sich das zweite Betätigungselement (224, 1552, 1652, 1752) in der ersten Position befindet, eine Drehung des ersten Betätigungselements (112, 212, 1112, 1555, 1655, 1755) auf die Entfaltungsanordnung (104, 304, 1904, 2004, 2304) übertragen wird, um die Entfaltungsanordnung (104, 304, 1904, 2004, 2304) zu veranlassen, sich zu drehen, und, wenn sich das zweite Betätigungselement (224, 1552, 1652, 1752) in der zweiten Position befindet, eine Drehung des ersten Betätigungselements (112, 212, 1112, 1555, 1655, 1755) die Hülse (320, 2020, 2223, 2323, 2327, 2427, 2527) veranlasst, sich in Längsrichtung relativ zu dem Handgriff (106, 206, 1106, 1906, 2006) zum Expandieren und Implantieren der Herzklappenprothese zu bewegen.

2. Kathetersystem (100, 1900, 2100) nach Anspruch 1,
wobei der Handgriff (106, 206, 1106, 1906, 2006) ein drittes Betätigungselement (211, 1140, 1240, 1340, 2040) umfasst, das, wenn es sich in einer ersten Position befindet, eine Bewegung des ersten Betätigungselements (112, 212, 1112, 1555, 1655, 1755) verhindert, wenn sich das zweite Betätigungselement (224, 1552, 1652, 1752) in der ersten Position befindet, und, wenn es sich in einer zweiten Position befindet, eine Bewegung des ersten Betätigungselements (112, 212, 1112, 1555, 1655, 1755) gestattet, wenn sich das zweite Betätigungselement (224, 1552, 1652, 1752) in der ersten Position befindet.

3. Kathetersystem (100, 1900, 2100) nach Anspruch 2,
wobei der Handgriff (106, 206, 1106, 1906, 2006) ein viertes Betätigungselement (110, 210, 1110, 1910) umfasst, das dazu eingerichtet ist, sich relativ zu dem Handgriffkörper (108, 208, 1108, 1408, 1908) und unabhängig von dem ersten Betätigungselement (112, 212, 1112, 1555, 1655, 1755) zu drehen, wobei das vierte Betätigungselement (110, 210, 1110, 1910) mit einem distalen Abschnitt des länglichen Schafts (102, 202, 302, 402, 1102, 1902, 2003, 2332) verbunden und dazu eingerichtet ist, den länglichen Schaft (102, 202, 302, 402, 1102, 1902, 2003, 2332) zu veranlassen, sich auszulenken.

4. Kathetersystem (100, 1900, 2100) nach Anspruch 1,
wobei, wenn sich das zweite Betätigungselement (224, 1552, 1652, 1752) in der zweiten Position befindet, eine Drehung des ersten Betätigungselements (112, 212, 1112, 1555, 1655, 1755) die Entfaltungsanordnung (104, 304, 1904, 2004, 2304) nicht veranlasst, sich zu drehen, und, wenn sich das zweite Betätigungselement (224, 1552, 1652, 1752) in der ersten Position befindet, eine Drehung des ersten Betätigungselements (112, 212, 1112, 1555, 1655, 1755) die Hülse (320, 2020, 2223, 2323, 2327, 2427, 2527) nicht veranlasst, sich in Längsrichtung relativ zu dem Handgriff (106, 206, 1106, 1906, 2006) zu bewegen.

5. Kathetersystem (100, 1900, 2100) nach Anspruch 1,
die Entfaltungsanordnung (104, 304, 1904, 2004, 2304) ferner umfassend eine zweite Hülse (312, 902, 2008, 2220) proximal zu der Hülse (320, 2020, 2223, 2323, 2327, 2427, 2527) und gekoppelt mit dem länglichen Schaft (102, 202, 302, 402, 1102, 1902, 2003, 2332) und einen Ankerträger (322, 2022, 2222, 2322, 2422, 2522), der innerhalb der zweiten Hülse (312, 902, 2008, 2220) und in mechanischer Kommunikation mit der Hülse (320, 2020, 2223, 2323, 2327, 2427, 2527) positioniert ist.

6. Kathetersystem (100, 1900, 2100) nach Anspruch 5,
wobei der Ankerträger (322, 2022, 2222, 2322, 2422, 2522) dazu eingerichtet ist, einen proximalen Abschnitt der Klappenprothese aufzunehmen, und der Ankerträger (322, 2022, 2222, 2322, 2422, 2522) und die zweite Hülse (312, 902, 2008, 2220) dazu eingerichtet sind, den proximalen Abschnitt der Herzklappenprothese in einem komprimierten Zustand zu halten.

7. Kathetersystem (100, 1900, 2100) nach Anspruch 5,
wobei eine Längsbewegung der Hülse (320, 2020, 2223, 2323, 2327, 2427, 2527) eine Längsbewegung des Ankerträgers (322, 2022, 2222, 2322, 2422, 2522) veranlasst.

8. Kathetersystem (100, 1900, 2100) nach Anspruch 1,
wobei, wenn sich das zweite Betätigungselement (224, 1552, 1652, 1752) in der ersten Position befindet, das erste Betätigungselement (112, 212, 1112, 1555, 1655, 1755) starr mit der Entfaltungsanordnung (104, 304, 1904, 2004, 2304) verbunden ist.

9. Kathetersystem (100, 1900, 2100) nach Anspruch 1,
wobei das zweite Betätigungselement (224, 1552, 1652, 1752) einen Vorsprung umfasst und, wenn sich das zweite Betätigungselement (224, 1552, 1652, 1752) in der ersten Position befindet, der Vorsprung des zweiten Betätigungselements (224, 1552, 1652, 1752) dazu eingerichtet ist, mit einem Schaft (1144, 1247, 1347, 1444, 1568, 1668, 1868) in Eingriff zu gelangen, der innerhalb des Handgriffs (106, 206, 1106, 1906, 2006) angeordnet und starr mit der Entfaltungsanordnung (104, 304, 1904, 2004, 2304) verbunden ist.

10. Kathetersystem (100, 1900, 2100) nach Anspruch 9,
wobei der Schaft (1144, 1247, 1347, 1444, 1568, 1668, 1868) mit Gewinde versehen ist und ein Querschnitt des Schafts (1144, 1247, 1347, 1444, 1568, 1668, 1868) mindestens einen rechten Winkel umfasst.

11. Kathetersystem (100, 1900, 2100) nach Anspruch 10,
wobei der Handgriff (106, 206, 1106, 1906, 2006) ferner ein drittes Betätigungselement (211, 1140, 1240, 1340, 2040) umfasst, das dazu eingerichtet ist, mit einem oder mehreren Gewindegängen (910, 1566, 1669) des Schafts (1144, 1247, 1347, 1444, 1568, 1668, 1868) und dem mindestens einen rechten Winkel zusammenzuwirken, um die Entfaltungsanordnung (104, 304, 1904, 2004, 2304) selektiv an einer axialen und rotatorischen Bewegung zu hindern.

12. Kathetersystem (100, 1900, 2100) nach Anspruch 11,
das dritte Betätigungselement (211, 1140, 1240, 1340, 2040) umfassend einen niederdrückbaren Körper mit einem zentralen Kanal und ein geripptes Rad (1244), das innerhalb des zentralen Kanals angeordnet und dazu eingerichtet ist, den Schaft (1144, 1247, 1347, 1444, 1568, 1668, 1868) aufzunehmen und sich mit diesem zu drehen, und wobei das dritte Betätigungselement (211, 1140, 1240, 1340, 2040) dazu eingerichtet ist, einer Drehung des gerippten Rads (1244) in einer verriegelten Position zu widerstehen.

## Revendications

1. Système de cathéter (100, 1900, 2100) destiné à l'implantation d'une valve cardiaque prothétique, le système de cathéter (100, 1900, 2100) comprenant:
- une tige allongée (102, 202, 302, 402, 1102, 1902, 2003, 2332) comprenant une région proximale et une région distale;
- un ensemble de déploiement (104, 304, 1904, 2004, 2204, 2304) situé au niveau de la région distale de la tige allongée (102, 202, 302, 402, 1102, 1902, 2003, 2332), l'ensemble de déploiement (104, 304, 1904, 2004, 2304) étant dimensionné et formé pour être avancé jusqu'à un site d'implantation situé au niveau d'un site de valve cardiaque native avec la valve cardiaque prothétique dans un état replié, l'ensemble de déploiement (104, 304, 1904, 2004, 2304) comprenant un manchon (320, 2020, 2223, 2323, 2327, 2427, 2527) configuré pour maintenir au moins une partie de la valve cardiaque prothétique dans l'état replié à l'intérieur du manchon (320, 2020, 2223, 2323, 2327, 2427, 2527) pendant l'acheminement; et
- une poignée (106, 206, 1106, 1906, 2006) disposée au niveau d'une région proximale de la tige allongée (102, 202, 302, 402, 1102, 1902, 2003, 2332), la poignée (106, 206, 1106, 1906, 2006) comprenant un corps de poignée (108, 208, 1108, 1408, 1908), un premier actionneur (112, 212, 1112, 1555, 1655, 1755) configuré pour être tourné par rapport au corps de poignée (108, 208, 1108, 1408, 1908), et un deuxième actionneur (224, 1552, 1652, 1752) configuré pour effectuer une transition entre une première position et une deuxième position, dans lequel, lorsque le deuxième actionneur (224, 1552, 1652, 1752) est dans la première position, une rotation du premier actionneur (112, 212, 1112, 1555, 1655, 1755) est transmise à l'ensemble de déploiement (104, 304, 1904, 2004, 2304) de manière à provoquer la rotation de l'ensemble de déploiement (104, 304, 1904, 2004, 2304), et lorsque le deuxième actionneur (224, 1552, 1652, 1752) est dans la deuxième position, une rotation du premier actionneur (112, 212, 1112, 1555, 1655, 1755) provoque le déplacement longitudinal du manchon (320, 2020, 2223, 2323, 2327, 2427, 2527) par rapport à la poignée (106, 206, 1106, 1906, 2006) afin de déployer et d'implanter la valve cardiaque prothétique.

2. Système de cathéter (100, 1900, 2100) selon la revendication 1, dans lequel la poignée (106, 206, 1106, 1906, 2006) comprend un troisième actionneur (211, 1140, 1240, 1340, 2040) qui, lorsqu'il est dans une première position, empêche un mouvement du premier actionneur (112, 212, 1112, 1555, 1655, 1755) lorsque le deuxième actionneur (224, 1552, 1652, 1752) est dans la première position et, lorsqu'il est dans une deuxième position, permet un mouvement du premier actionneur (112, 212, 1112, 1555, 1655, 1755) lorsque le deuxième actionneur (224, 1552, 1652, 1752) est dans la première position.

3. Système de cathéter (100, 1900, 2100) selon la revendication 2, dans lequel la poignée (106, 206, 1106, 1906, 2006) comprend un quatrième actionneur (110, 210, 1110, 1910) configuré pour tourner par rapport au corps de poignée (108, 208, 1108, 1408, 1908) et indépendamment du premier actionneur (112, 212, 1112, 1555, 1655, 1755), le quatrième actionneur (110, 210, 1110, 1910) étant relié à une partie distale de la tige allongée (102, 202, 302, 402, 1102, 1902, 2003, 2332) et configuré pour provoquer la flexion de la tige allongée (102, 202, 302, 402, 1102, 1902, 2003, 2332).

4. Système de cathéter (100, 1900, 2100) selon la revendication 1, dans lequel, lorsque le deuxième actionneur (224, 1552, 1652, 1752) est dans la deuxième position, une rotation du premier actionneur (112, 212, 1112, 1555, 1655, 1755) ne provoque pas la rotation de l'ensemble de déploiement (104, 304, 1904, 2004, 2304), et lorsque le deuxième actionneur (224, 1552, 1652, 1752) est dans la première position, une rotation du premier actionneur (112, 212, 1112, 1555, 1655, 1755) ne provoque pas le déplacement longitudinal du manchon (320, 2020, 2223, 2323, 2327, 2427, 2527) par rapport à la poignée (106, 206, 1106, 1906, 2006).

5. Système de cathéter (100, 1900, 2100) selon la revendication 1, dans lequel l'ensemble de déploiement (104, 304, 1904, 2004, 2304) comprend en outre un deuxième manchon (312, 902, 2008, 2220) proximal par rapport au manchon (320, 2020, 2223, 2323, 2327, 2427, 2527) et couplé à la tige allongée (102, 202, 302, 402, 1102, 1902, 2003, 2332), ainsi qu'un support d'ancrage (322, 2022, 2222, 2322, 2422, 2522) positionné à l'intérieur du deuxième manchon (312, 902, 2008, 2220) et en liaison mécanique avec le manchon (320, 2020, 2223, 2323, 2327, 2427, 2527).

6. Système de cathéter (100, 1900, 2100) selon la revendication 5, dans lequel le support d'ancrage (322, 2022, 2222, 2322, 2422, 2522) est configuré pour recevoir une partie proximale de la valve prothétique, et le support d'ancrage (322, 2022, 2222, 2322, 2422, 2522) ainsi que le deuxième manchon (312, 902, 2008, 2220) sont configurés pour maintenir la partie proximale de la valve cardiaque prothétique dans un état comprimé.

7. Système de cathéter (100, 1900, 2100) selon la revendication 5, dans lequel un déplacement longitudinal du manchon (320, 2020, 2223, 2323, 2327, 2427, 2527) entraîne un déplacement longitudinal du support d'ancrage (322, 2022, 2222, 2322, 2422, 2522).

8. Système de cathéter (100, 1900, 2100) selon la revendication 1, dans lequel, lorsque le deuxième actionneur (224, 1552, 1652, 1752) est dans la première position, le premier actionneur (112, 212, 1112, 1555, 1655, 1755) est relié de manière rigide à l'ensemble de déploiement (104, 304, 1904, 2004, 2304).

9. Système de cathéter (100, 1900, 2100) selon la revendication 1, dans lequel le deuxième actionneur (224, 1552, 1652, 1752) comprend une saillie et, lorsque le deuxième actionneur (224, 1552, 1652, 1752) est dans la première position, la saillie du deuxième actionneur (224, 1552, 1652, 1752) est configurée pour venir en prise avec une tige (1144, 1247, 1347, 1444, 1568, 1668, 1868) disposée à l'intérieur de la poignée (106, 206, 1106, 1906, 2006) et reliée de manière rigide à l'ensemble de déploiement (104, 304, 1904, 2004, 2304).

10. Système de cathéter (100, 1900, 2100) selon la revendication 9, dans lequel la tige (1144, 1247, 1347, 1444, 1568, 1668, 1868) est filetée et une section transversale de la tige (1144, 1247, 1347, 1444, 1568, 1668, 1868) comprend au moins un angle droit.

11. Système de cathéter (100, 1900, 2100) selon la revendication 10, dans lequel la poignée (106, 206, 1106, 1906, 2006) comprend en outre un troisième actionneur (211, 1140, 1240, 1340, 2040) configuré pour coopérer avec un ou plusieurs filetages (910, 1566, 1669) de la tige (1144, 1247, 1347, 1444, 1568, 1668, 1868) et l'au moins un angle droit afin d'empêcher sélectivement tout mouvement axial et toute rotation de l'ensemble de déploiement (104, 304, 1904, 2004, 2304).

12. Système de cathéter (100, 1900, 2100) selon la revendication 11, dans lequel le troisième actionneur (211, 1140, 1240, 1340, 2040) comprend un corps pouvant être enfoncé et présentant un canal central, ainsi qu'une roue nervurée (1244) disposée à l'intérieur du canal central et configurée pour recevoir la tige (1144, 1247, 1347, 1444, 1568, 1668, 1868) et tourner avec celle-ci, et dans lequel le troisième actionneur (211, 1140, 1240, 1340, 2040) est configuré pour résister à la rotation de la roue nervurée (1244) dans une position verrouillée.
